(19) Europäisches Patentamt
European Patent Office
Office européen des brevets

(11) EP 0 800 504 B1

(12) EUROPEAN PATENT SPECIFICATION

(45) Date of publication and mention of the grant of the patent: **01.12.1999 Bulletin 1999/48**

(21) Application number: **95944221.1**

(22) Date of filing: **21.12.1995**

(51) Int Cl.[6]: **C07C 57/50**, C07D 223/00, C07D 309/00, C07D 215/00, C07D 265/00, A61K 31/19

(86) International application number: **PCT/US95/16842**

(87) International publication number: **WO 96/20914 (11.07.1996 Gazette 1996/31)**

(54) **TRICYCLIC RETINOIDS, METHODS FOR THEIR PRODUCTION AND USE**

TRIZYCLISCHE RETINOIDVERBINDUNGEN VERFAHREN ZU IHRER HERSTELLUNG UND VERWENDUNG

RETINOIDES TRICYCLIQUES ET LEURS PROCEDES DE PRODUCTION ET D'UTILISATION

(84) Designated Contracting States: **AT BE CH DE ES FR GB IT LI NL**

(30) Priority: **30.12.1994 US 366630**
**07.06.1995 US 475397**
**07.06.1995 US 472127**
**07.06.1995 US 475514**

(43) Date of publication of application: **15.10.1997 Bulletin 1997/42**

(60) Divisional application: **99105682.1 / 0 933 351**
**99105654.0 / 0 933 350**

(73) Proprietor: **LIGAND PHARMACEUTICALS INCORPORATED**
**San Diego, California 92121 (US)**

(72) Inventors:
- **HWANG, Chan, Kou**
  **Boulder, CO 80304 (US)**
- **WHITE, Steven, K.**
  **San Diego, CA 92129 (US)**
- **BENNANI, Youssef, L.**
  **La Jolla, CA 92037 (US)**
- **CANAN KOCH, Stacie, S.**
  **San Diego, CA 92116 (US)**
- **BADEA, Beth, Ann**
  **San Diego, CA 92127 (US)**
- **HEBERT, Jonathan, J.**
  **Mission Viejo, CA 92692 (US)**
- **FARMER, Luc, J.**
  **La Jolla, CA 92037 (US)**
- **NADZAN, Alex, M.**
  **San Diego, CA 92130 (US)**

(74) Representative: **Fürniss, Peter, Dr.**
**Winter, Brandl, Fürniss, Hübner, Röss, Kaiser, Polte, Kindermann Partnerschaft**
**Patent- und Rechtsanwaltskanzlei**
**85354 Freising (DE)**

(56) References cited:
- **No relevant documents disclosed**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 800 504 B1

Printed by Jouve, 75001 PARIS (FR)

## Description

The present invention relates to retinoid compounds having activity for retinoic acid receptors and retinoid X receptors, and to methods for the production and therapeutic use of such compounds.

### Background of the Invention

The vitamin A metabolite, retinoic acid, has long been recognized to induce a broad spectrum of biological effects. In addition, a variety of structural analogues of retinoic acid have been synthesized that also have been found to be bioactive. Some, such as Retin-A® and Accutane®, have found utility as therapeutic agents for the treatment of various pathological conditions. In addition, synthetic retinoids have been found to mimic many of the pharmacological actions of retinoic acid.

Medical professionals have become very interested in the therapeutic applications of retinoids. Among their uses approved by the FDA is the treatment of severe forms of acne and psoriasis. A large body of evidence also exists that these compounds can be used to arrest and, to an extent, reverse the effects of skin damage arising from prolonged exposure to the sun. Other evidence exists that these compounds may be useful in the treatment and prevention of a variety of cancerous and pre-cancerous conditions, such as melanoma. cervical cancer, some forms of leukemia, oral leukoplakia and basal and squamous cell carcinomas. Retinoids have also shown an ability to be efficacious in treating and preventing diseases of the eye, cardiovascular system, immune system, skin, respiratory and digestive tracts, and as agents to facilitate wound healing and modulate programmed cell death (apoptosis).

Major insight into the molecular mechanism of retinoic acid signal transduction was gained in 1988, when a member of the steroid/thyroid hormone intracellular receptor superfamily was shown to transduce a retinoic acid signal. Evans, *Science,* 240:889-95 (1988); Giguere et al., *Nature,* 330:624-29 (1987); Petkovich *et al., Nature,* 330: 444-50 (1987). It is now known that retinoids regulate the activity of two distinct intracellular receptor subfamilies; the Retinoic Acid Receptors (RARs) and the Retinoid X Receptors (RXRs), including their isoforms, RARα, β, γ and RXRα, β, γ. In this regard, an endogenous low-molecular-weight ligand which modulates the transcriptional activity of the RARs is all-*trans*-retinoic acid (ATRA), while an endogenous ligand for the RXRs is 9-*cis* retinoic acid (9-*cis*). Heyman *et al., Cell*, 68:397-406 (1992) and Levin *et al. Nature*, 355:359-61(1992).

Although both the RARs and RXRs respond to ATRA *in vivo,* due to the *in vivo* conversion of some of the ATRA to 9-*cis,* the receptors differ in several important aspects. First, the RARs and RXRs are significantly divergent in primary structure *(e.g.,* the ligand binding domains of RARα and RXRα have only 27% amino acid identity). These structural differences are reflected in the different relative degrees of responsiveness of RARs and RXRs to various vitamin A metabolites and synthetic retinoids. In addition, distinctly different patterns of tissue distribution are seen for RARs and RXRs. For example, in contrast to the RARs, which are not expressed at high levels in the visceral tissues, RXRα mRNA has been shown to be most abundant in the liver, kidney, lung, muscle. and intestine. Finally, the RARs and RXRs have different target gene specificity. For example, response elements have recently been identified in the cellular retinal binding protein type II (CRBPII) and Apolipoprotein AI genes which confer responsiveness to RXR, but not RAR. Furthermore, RAR has also been recently shown to repress RXR-mediated activation through the CRBPII RXR response element (Manglesdorf *et al., Cell,* 66:555-61 (1991)). These data indicate that two retinoic acid responsive pathways are not simply redundant, but instead manifest a complex interplay.

In view of the related, but clearly distinct, nature of these receptors, retinoids which are more selective for the RAR subfamily or the RXR subfamily would be of great value for selectively controlling processes mediated by one or more of the RAR or RXR isoforms, and would provide the capacity for independent control of the physiologic processes mediated by the RARs or RXRs. In addition, pan-agonist retinoids that activate one or more isoforms of both the RARs and RXRs would also be valuable for controlling processes mediated by both of these subfamilies of retinoid receptors. Furthermore, retinoids which preferentially affect one or more but not all of the receptor isoforms also offer the possibility of increased therapeutic efficacy and reduced side effect profiles when used for therapeutic applications.

Various tricyclic compounds, the majority of which are beta-substituted on the C ring, have been disclosed to have retinoid activity, including those compounds disclosed in H. Kagechika et al., "Retinobenzoic Acids. 2. Structure-Activity Relationship of Chalcone-4-carboxylic Acids and Flavone-4'-carboxylic Acids", 32 *J. Med Chem.,* 834 (1989); H. Kagechika et al., "Retinobenzoic Acids. 3. Structure-Activity Relationships of Retinoidal Azobenzene-4-carboxylic Acids and Stilbene-4-carboxylic Acids", 32 *J. Med Chem.,* 1098 (1989); H. Kagechika et al., "Retinobenzoic Acids. 4. Conformation of Aromatic Amides with Retinoidal Activity. Importance of *trans*-Amide Structure for the Activity", 32 *J. Med Chem.,* 2292 (1989); M.I. Dawson et al., "Effect of Structural Modifications in the C7-C11 Region of the Retinoid Skeleton on Biological Activity in a Series of Aromatic Retinoids", 32 *J. Med. Chem.,* 1504 (1989) and U.S. Patent Nos. 4,831,052, 4,874,747, 4,925,979, 5,004,730, 5,124, 473 and Re 33,533.

## Summary of the Invention

The present invention provides novel tricyclic retinoid compounds that have selective activity on RARs and RXRs or pan-agonist activity on one or more of each of the RAR and RXR isoforms. The present invention also provides pharmaceutical compositions incorporating these novel tricyclic compounds and methods for the therapeutic use of such compounds and pharmaceutical compositions.

These and various other advantages and features of novelty which characterize the invention are pointed out with particularity in the claims annexed hereto and forming a part hereof. However, for a better understanding of the invention, its advantages, and objects obtained by its use, reference should be had to the accompanying drawings and descriptive matter, in which there is illustrated and described preferred embodiments of the invention.

## Definitions

In accordance with the present invention and as used herein, the following terms are defined with the following meanings, unless explicitly stated otherwise.

The term alkyl refers to straight-chain, branched-chain, cyclic structures, and combinations thereof.

The term aryl refers to an optionally substituted six-membered aromatic ring.

The term heteroaryl refers to an optionally substituted five-membered or six-membered heterocyclic ring containing one or more heteroatoms selected from the group consisting of oxygen, nitrogen and sulfur.

The terms retinoid or retinoids refer to compound(s) that bind and/or activate one or more retinoid receptors, thereby affecting the transcriptional activity of a target gene to which the activated receptor and compound complex binds.

The term pan-agonist refers to a retinoid that activates at least one member of both the RAR subfamily (i.e., RARα, RARβ, or RARγ) and the RXR subfamily (i.e., RXRα, RXRβ, or RXRγ). Preferably such pan-agonist retinoids activate all members of both the RAR and RXR subfamilies of retinoid receptors.

## Detailed Description of Embodiments of the Invention

In accordance with a first aspect of the present invention, we have developed tricyclic retinoid compounds of the formula:

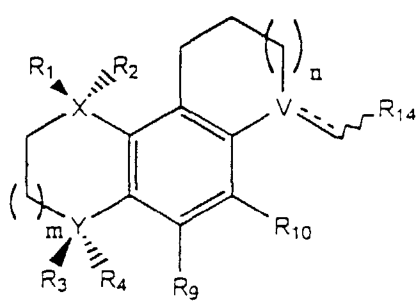

(III)

wherein,

$R_1$ through $R_4$ each independently are hydrogen, a $C_1$ - $C_6$ alkyl or a $C_7$ - $C_{15}$ arylalkyl;
$R_9$ and $R_{10}$ each independently are hydrogen, a $C_1$ - $C_6$ alkyl, F, Cl, Br, $NR_{11}R_{12}$, $NO_2$ or $OR_{13}$, where $R_{11}$ and $R_{12}$ each independently are hydrogen, a $C_1$ - $C_8$ alkyl, a $C_7$ - $C_{15}$ arylalkyl, a $C_1$ - $C_8$ acyl, provided that only one of $R_{11}$ or $R_{12}$ can be acyl, or $R_{11}$ and $R_{12}$ taken together are a $C_3$ - $C_6$ cycloalkyl, and where $R_{13}$ is hydrogen or a $C_1$ - $C_8$ alkyl or a $C_7$ - $C_{15}$ arylalkyl;
$R_{14}$ represents:

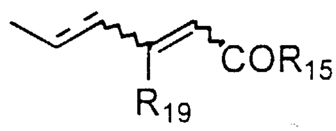 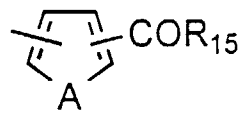 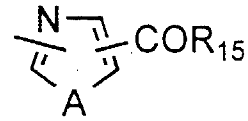

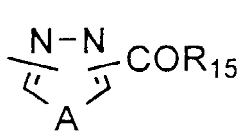 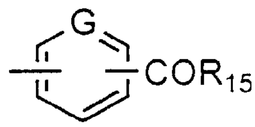 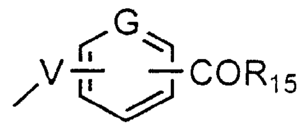

where $R_{15}$ is $OR_{16}$ or $NR_{17}R_{18}$, with $R_{16}$ being hydrogen, a $C_1$ - $C_6$ alkyl or a $C_7$ - $C_{15}$ arylalkyl, and with $R_{17}$ and $R_{18}$ each independently being hydrogen, a $C_1$ - $C_6$ alkyl, a $C_7$ - $C_{15}$ arylalkyl, aryl, ortho-, meta-, or para-substituted hydroxyaryl, or taken together are a $C_3$ - $C_6$ cycloalkyl, provided that $R_{18}$ must be hydrogen when $R_{17}$ is aryl or hydroxyaryl, $R_{19}$ is a $C_1$ - $C_5$ alkyl, and A is O, S or $NR_{20}$, where $R_{20}$ is a hydrogen, $C_1$ - $C_6$ alkyl or a $C_7$ - $C_{15}$ arylalkyl;

X and Y each independently represent C, O, S, N, SO or $SO_2$, provided, however, that when X or Y are O, S, SO or $SO_2$, then either $R_1$ and $R_2$ or $R_3$ and $R_4$ respectively do not exist, and further provided, that when X or Y is N, then one each of $R_1$ and $R_2$ or $R_3$ and $R_4$ respectively, do not exist;

V is C or N, provided, however, that when V is N, then no double bond exists adjacent to V;

G is C or N;

m is 0 or 1 carbon atoms;

n is 0, 1 or 2 carbon atoms;

the dashed lines in the structures represent optional double bonds; and

the wavy lines represent olefin bonds that are either in the *cis* (Z) or *trans* (E) configuration.

The compounds of the present invention also include all pharmaceutically acceptable salts, as well as esters and amides. Preferably, such salts, esters and amides, will be formed at the $R_{15}$ and $R_{16}$ positions. As used in this disclosure, pharmaceutically acceptable salts include, but are not limited to: pyridine, ammonium, piperazine, diethylamine, nicotinamide, formic, urea, sodium, potassium, calcium, magnesium, zinc, lithium, cinnamic, methylamino, methanesulfonic, picric, tartaric, tnethylamino, dimethylamino, and tris(hydoxymethyl)aminomethane. Additional pharmaceutically acceptable salts are known to those skilled in the art.

The compounds of the present invention are particularly useful in the treatment of skin-related diseases, including, without limitation, actinic keratoses, arsenic keratoses, inflammatory and non-inflammatory acne, psoriasis, ichthyoses and other keratinization and hyperproliferative disorders of the skin, eczema, atopic dermatitis, Darriers disease, lichen planus, prevention and reversal of glucocorticoid damage (steroid atrophy), as a topical antimicrobial, as skin pigmentation agents and to treat and reverse the effects of age and photo damage to the skin. The compounds are also useful for the prevention and treatment of cancerous and pre-cancerous conditions, including, premalignant and malignant hyperproliferative diseases such as cancers of the breast, skin, prostate, cervix, uterus, colon, bladder, esophagus, stomach, lung, larynx, oral cavity, blood and lymphatic system, metaplasias, dysplasias, neoplasias, leukoplakias and papillomas of the mucous membranes and in the treatment ofKaposis sarcoma. In addition, the present compounds can be used as agents to treat diseases of the eye, including, without limitation, proliferative vitreoretinopathy (PVR), retinal detachment, dry eye and other corneopathies, as well as in the treatment and prevention of various cardiovascular diseases, including, without limitation, diseases associated with lipid metabolism such as dyslipidemias, prevention of restenosis and as an agent to increase the level of circulating tissue plasminogen activator (TPA). Other uses for the compounds of the present invention include the prevention and treatment of conditions and diseases associated with human papilloma virus (HPV), including warts and genital warts, various inflammatory diseases such as pulmonary fibrosis, ileitis, colitis and Krohn's disease, neurodegenerative diseases such as Alzheimer's disease, Parkinson's disease and Amyotrophic Lateral Sclerosis (ALS), improper pituitary function, including insufficient production of growth hormone, modulation of apoptosis, including both the induction of apoptosis and inhibition of T-Cell activated apoptosis, restoration of hair growth, including combination therapies with the present compounds and other agents such as Minoxidil®, diseases associated with the immune system, including use of the present compounds as immunosuppressants and immunostimulants, modulation of organ transplant rejection and facilitation of wound healing, including modulation of chelosis. It will also be understood by those skilled in the art that the retinoid compounds of the present invention will prove useful in any therapy in which retinoids, including RAR selective retinoids, RXR selective retinoids, and pan-agonist retinoids will find application.

Furthermore, it will be understood by those skilled in the art that the compounds of the present invention, including pharmaceutical compositions and formulations containing these compounds, can be used in a wide variety of combination therapies to treat the conditions and diseases described above. Thus, the compounds of the present invention can be used in combination with other therapies, including, without limitation, chemotherapeutic agents such as cytostatic and cytotoxic agents, immunological modifiers such as interferons, interleukins, growth hormones and other cytokines, hormone therapies, surgery and radiation therapy.

Representative retinoids of the present invention include, without limitation, (2E,4E)-3-methyl-6-[(E)-2,3,6,7,8,9-hexahydro-6,6,9,9-tetramethyl-1H-cyclopenta[a]naphthalen-3-ylidene]hexa-2,4-dienoic acid; ethyl (2E, 4E)-3-methyl-6-(2,3,6,7,8,9-hexahydro-6,6,9,9-tetramethyl-1H-cyclopenta[a]naphthalen-3yl]hexa-2,4-dienoate; (2E, 4E)-3-methyl-6-(2,3,6,7,8,9-hexahydro-6,6,9,9-tetramethyl-1H-cyclopenta[a]naphthalen-3-yl)hexa-2,4-dienoic acid; ethyl (2E,4E)-3-methyl-6-[(Z)-1,2,3,4,7,8,9-heptahydro-7,7,9,9-tetramethylcyclopenta[f]naphthalen-4-ylidene]hexa-2,4-dienoate; (2E,4E)-3-methyl-6-[(Z)-1,2,3,4,7,8,9-heptahydro-7,7,9,9-tetramethylcyclopenta[f]naphthalen-4-ylidene]hexa-2,4-dienoic acid; ethyl(2E,4E)-3-methyl-6-(1,2,3,4,7,8,9,10-octahydro-7,7,10,10-tetramethylbenzo[f]quinolin-4-yl)hexa-2,4-dienoate; and (2E,4E)-3-methyl-6-(1,2,3,4,7,8,9,10-octahydro-7,7,10,10-tetramethylbenzo[f]quinolin-4-yl)hexa-2,4-dienoic acid.

The compounds of the present invention can be obtained by modification of the compounds disclosed or by a total synthesis approach, by techniques known to those skilled in the art. In this regard, the synthesis of the compounds of the present invention often follow established retinoid synthesis schemes and techniques as described in M.I. Dawson and W.H. Okamura, "Chemistry and Biology of Synthetic Retinoids", Chapters 3, 8, 14 and 16, CRC Press, Inc., Florida (1990); M.I. Da$_w$son and P.D. Hobbs, *The Synthetic Chemistry of Retinoids, In* Chapter 2: "The Retinoids, Biology, Chemistry and Medicine", M.B. Sporn et al., Eds. (2nd ed.), Raven Press, New York, New York, pp. 5-178 (1994); R.S.H. Liu and A. E. Asato, "Photochemistry and Synthesis of Stereoisomers of Vitamin A," 40 *Tetrahedron,* 1931 (1984); 43 *Cancer Res.,* 5268 (1983); 15 *Eur. J. Med. Chem.,* 9 (1980); and U.S. Patent Nos. 4,326,055 and 4,578,498, the disclosures of which are herein incorporated by reference. The sequence of steps of the general schemes of synthesizing the compounds of the present invention are shown below. In addition, more detailed and illustrative synthetic schemes for specific compounds of the present invention will be found in the Examples included herein.

Scheme I: Synthesis of Compounds of Structure (I):

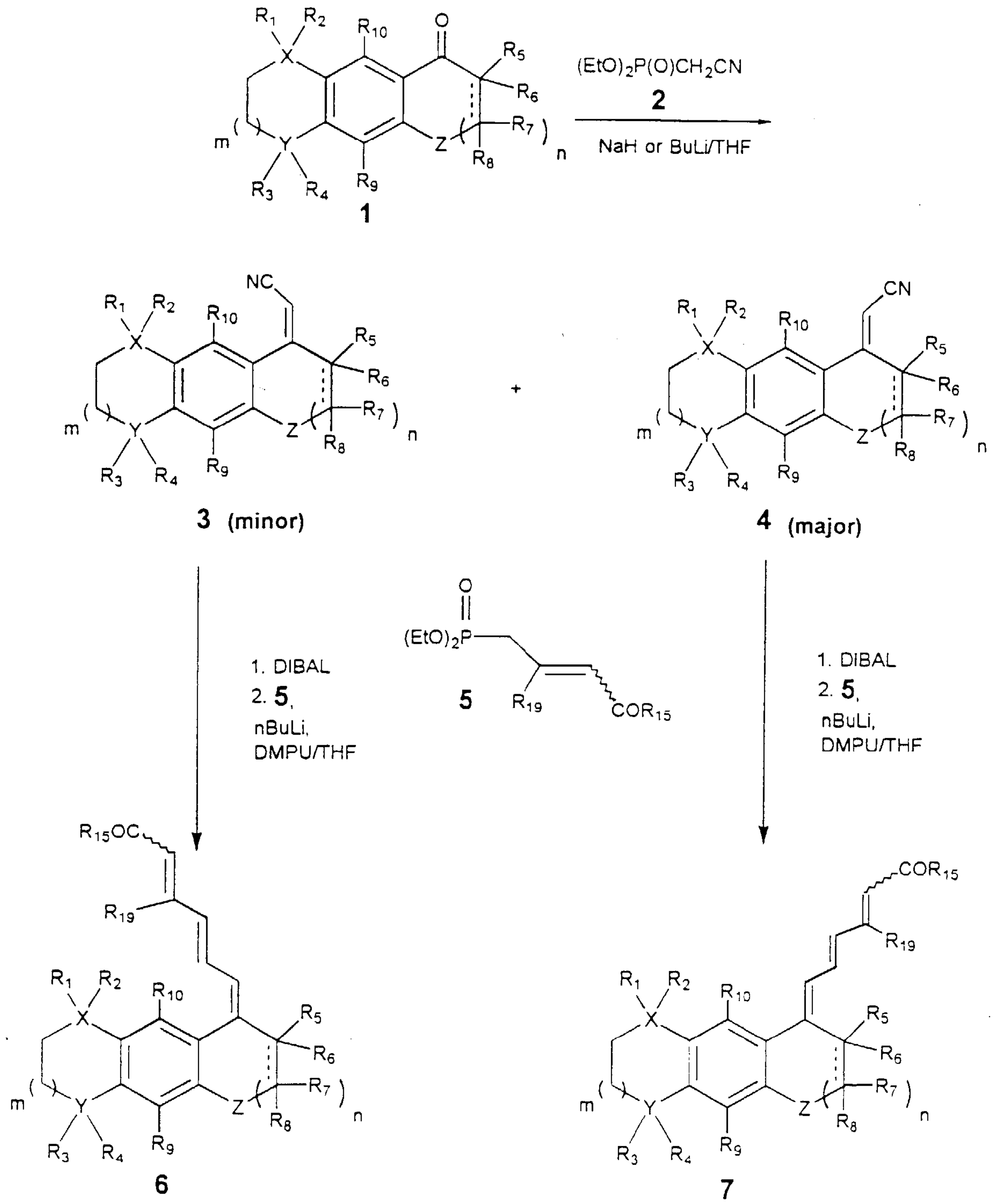


1

3 (minor) + 4 (major)

5

6

7

The tricyclic derivatives that is compounds of general structures **6** and **7**, may be prepared in accordance with reaction Scheme I. The starting materials for this sequence, ketones of general structure **1**, may be prepared from the appropriately substituted octahydroanthracene by oxidation with chromium trioxide in acetic acid at ambient temperature or with chromium trioxide in methylene chloride/pyridine at 0°C. Further, in accordance with this sequence of reactions tricyclic ketones of general structure **1** are condensed with the sodium or lithium salt of diethyl cyanomethylphosphonate in THF at reduced temperatures in a Horner-Wadsworth-Emmons olefination reaction to provide a mixture of the *cis*-cyano olefins **3** as the minor products and the *trans-cyano* olefins 4 as the major products. The olefinic products may be separated by silica gel flash column chromatography.

The cis-cyano olefins **3** are reduced with DIBAL at -78°C to give the intermediate enals. The solvent to be used in the reduction includes methylene chloride, hexanes, and THF. The product of the DIBAL reduction, the enal, is reacted with the lithium salt of diethyl 3-ethoxycarbonyl-2-methylprop-2-enylphosphonate (mixture of double bond isomers) in THF at reduced temperatures in a Horner-Wadsworth-Emmons olefination reaction to provide the tricyclic derivatives of general structure **6** where $R_{15}$ is ethyl. The olefination reaction is preferably conducted in the presence of 1,3-dimethyl-3,4,5,6-tetrahydro-2(1H)-pyrimidinone (DMPU). The acids and salts derived from structure **6** are readily obtainable from the corresponding esters. The ethyl esters may be hydrolyzed in an alkanol solvent at ambient temperature with about a three molar excess of base, for example, potassium hydroxide. Alternatively, the ethyl esters may be hydrolyzed in THF/water or acetone/water at ambient temperature with, for example, excess lithium hydroxide. The hydrolysis solution is acidified and the hydrolysate recovered by conventional means to give as the major product the (2E, 4E)-Z-ylidene tricyclic carboxylic acid derivatives of structure **6** where $R_{15}$ is OH. The minor (2Z, 4E)-Z-ylidene geometric isomers of general structure **6**, by-products of the olefination reaction, are readily isolated by silica gel chromatography of the hydrolysate mixture.

In an analogous fashion, in accordance with reaction Scheme I, the *trans-cyano* olefins **4** are transformed into the (2E, 4E)-E-ylidene and (2Z, 4E)-E-ylidene tricyclic derivatives **7** where $R_{15}$ is ethoxy and OH.

**Scheme II: Synthesis of Compounds of Structure (I):**

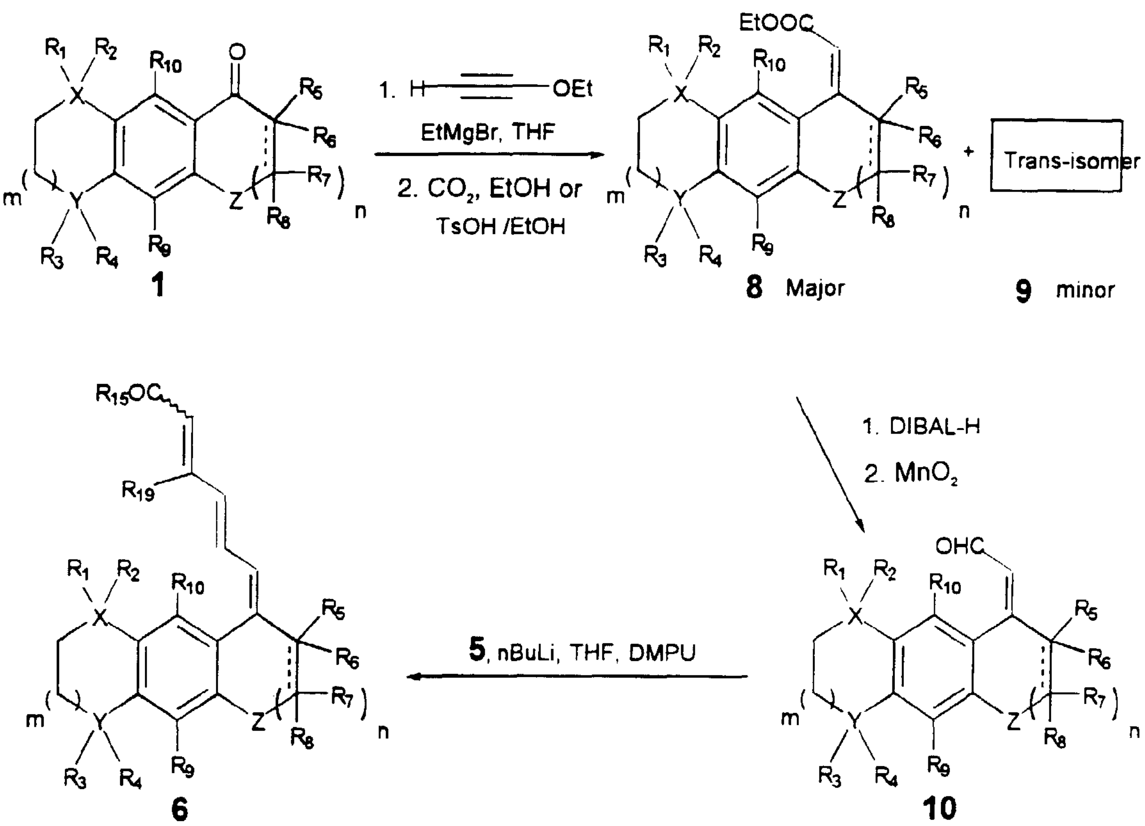


An alternative means for making the tricyclic derivatives of general structure **6** is in accordance with reaction Scheme II. Tricyclic ketones **1** are treated with ethoxy ethynyl magnesium bromide in THF at 0°C to ambient temperature. The resulting propargylic alcohols are isolated by typical extractive means, dissolved in ethanol and treated with carbon dioxide (gas) or, alternatively, a catalytic amount of *p*-toluensulfonic acid and/or camphor sulfonic acid to provide a mixture of the *cis*-olefinic esters **8** as the major product and the *trans*-olefinic esters of general structure **9** as the minor product. The olefinic products may be separated by silica gel flash column chromatography.

The *cis*-olefinic esters of general structure **8** are reduced with DIBAL at -78°C to give the intermediate allylic alcohol. The solvent to be used in the reduction includes methylene chloride, hexanes, and THF. The product of the

DIBAL reduction, the allylic alcohol, is oxidized with manganese dioxide in methylene chloride at ambient temperature to provide the *cis*-enals of general structure **10**. The enals are converted into tricyclic derivatives of general structure **6** by condensation with the phosphonate **5** by the same processes as those employed in the preparation process of Scheme I to produce tricycles of general structure **6** and **7**.

## Scheme III: Synthesis of Compounds of Structure (I):

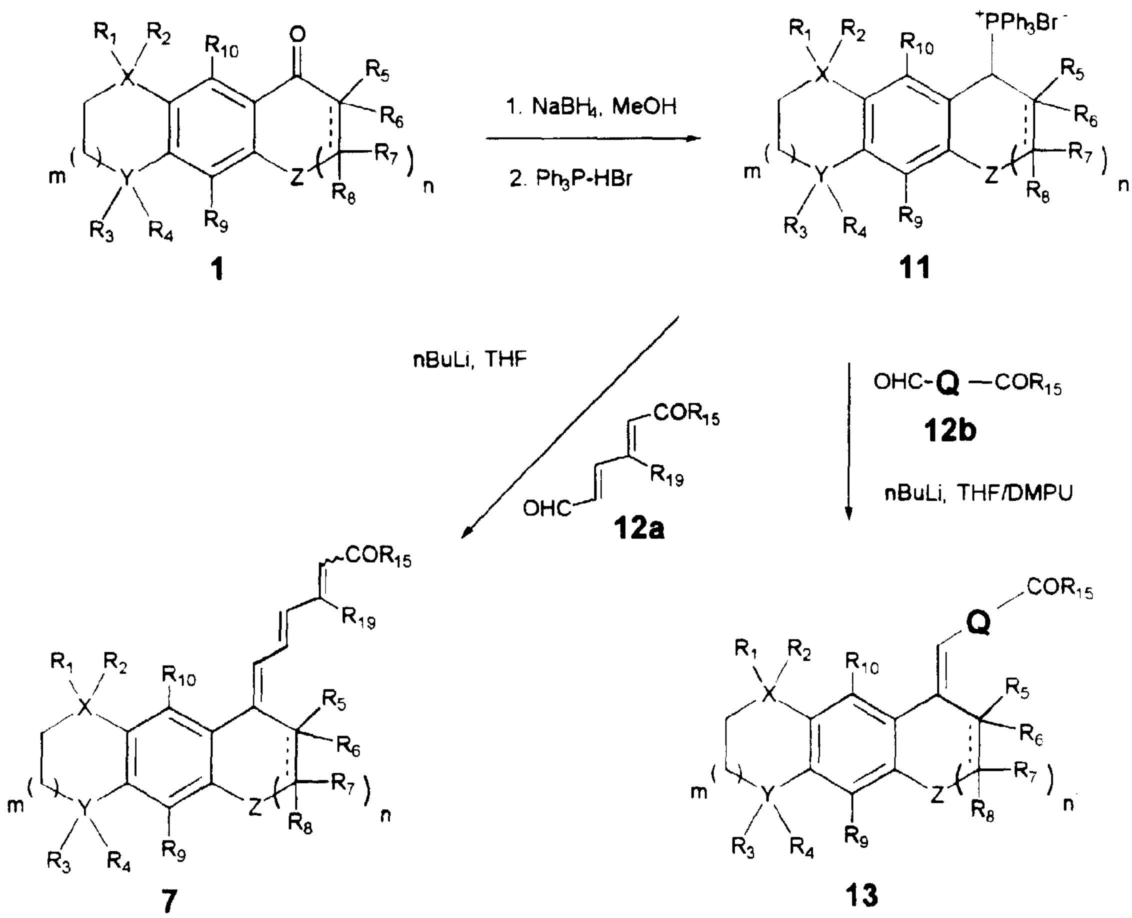


Q = 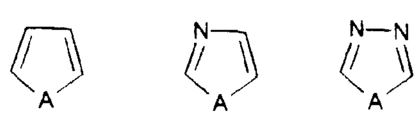

An alternative means for making the tricyclic derivatives of general structure **7** is in accordance with reaction Scheme III. The starting material for this sequence, ketones **1**, may be prepared from the appropriately substituted octahydroanthracenes by oxidation with chromium trioxide in acetic acid at ambient temperature or with chromium trioxide in methylene chloride/pyridine at low temperature. The tricyclic ketones are reduced with sodium borohydride in methanol at low temperature and the resultant benzylic alcohols are reacted with triphenylphosphine hydrobromide in methanol at elevated temperature to provide phosphonium salts of general structure **11**. Compounds **12a** where $R_{15}$

is ethyl and $R_{19}$ is methyl used for the following olefination reaction are prepared from commercially available (triphenylphosphoranylidene)acetaldehyde and ethyl 3-methyl-4-oxo-crotonate. A Horner-Wadsworth-Emmons olefination reaction in THF at reduced temperatures with the lithium salt of compounds **11** and compounds of general structure **12a** provide the tricyclic derivatives **7** where $R_{15}$ is ethoxy. The olefination reaction is preferably conducted in the presence of 1,3-dimethyl-3,4,5,6-tetrahydro-2(1H)-pyrimidinone (DMPU). The acids and salts derived from general structure **7** are readily obtainable from the corresponding esters by the same processes as those employed in the previously described process for tricycles of general structure **6**.

Compounds of general structure **13** may be prepared from the lithium salt of the phosphonium bromide of general structure **11** and aldehyde of general structure **12b** by the same olefination processes as those employed for the preparation of a compound of general structure **7**. The acids and salts derived from general structure **13** are readily obtainable from the corresponding esters by the same processes as those employed in the preparation process of Scheme I for tricycles of general structures **6** and **7**.

**Scheme IV: Synthesis of Compounds of Structure (I):**

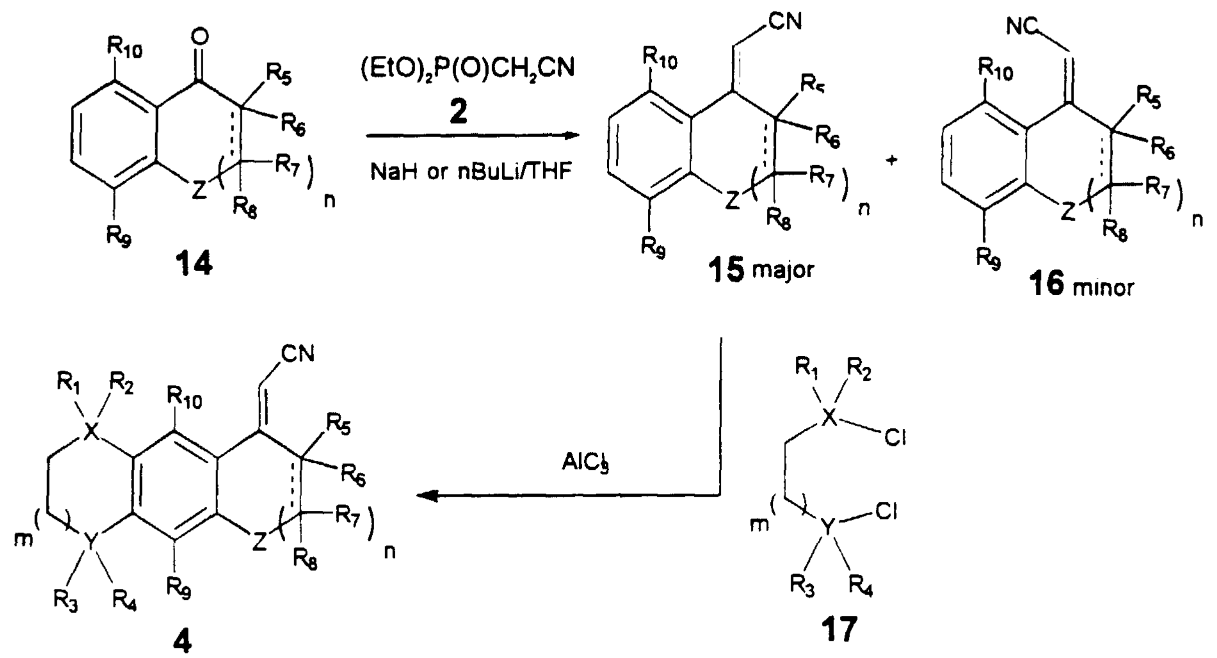

14, 15 major, 16 minor, 4, 17

An alternative means for making the intermediate tricyclic derivatives of general structure **4** is in accordance with reaction Scheme IV. The bicyclic ketones **14** are condensed with the sodium or lithium salt of diethyl cyanomethylphosphonate in THF at reduced temperatures in a Horner-Wadsworth-Emmons olefination reaction to provide a mixture of the *trans*-cyano olefins **15** as the major products and the *cis*-cyano olefins **16** as the minor products. The olefinic products may be separated by silica gel flash column chromatography The tricyclic derivatives of general structure **4** are prepared by aluminum trichloride catalyzed Friedel-Crafts alkylation/cyclization of 2,5-dichloro-2,5-dialkylhexanes **17** with the bicycles of general structure **15** and **16** in dichloromethane at ambient temperature.

**Scheme V: Synthesis of Compounds of Structure (II):**

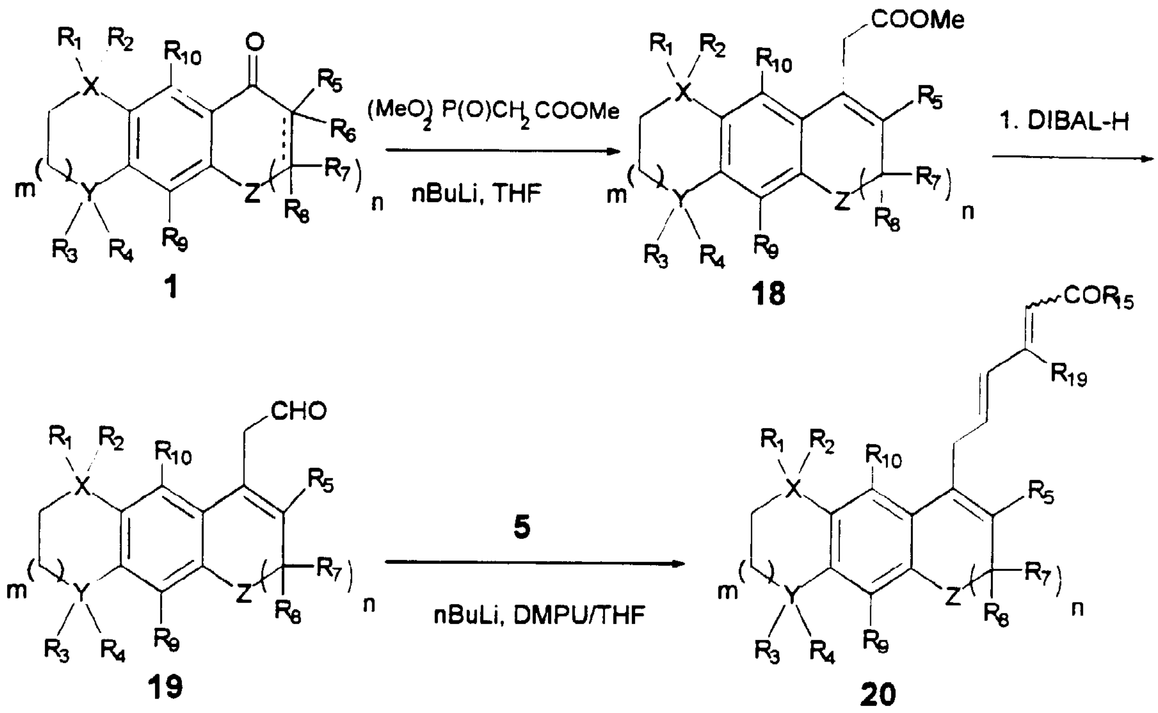


The tricyclic derivatives of general structure **20** can be prepared in accordance with reaction Scheme V. Tricyclic ketones **1** are condensed with the lithium salt of trimethyl phosphonoacetate in THF at elevated temperatures in a Horner-Wadsworth-Emmons olefination reaction to provide the β,γ-unsaturated esters **18**. The esters are reduced with one equivalent of DIBAL in methylene chloride at -78°C to provide aldehydes of general structure **19**. The aldehydes are converted into tricyclic derivatives **20** by condensation with the phosphonates **5** by the same processes as those employed in the process of Scheme I to produce tricycles of general structure **6** and **7**.

## Scheme VI: Synthesis of Compounds of Structure (II):

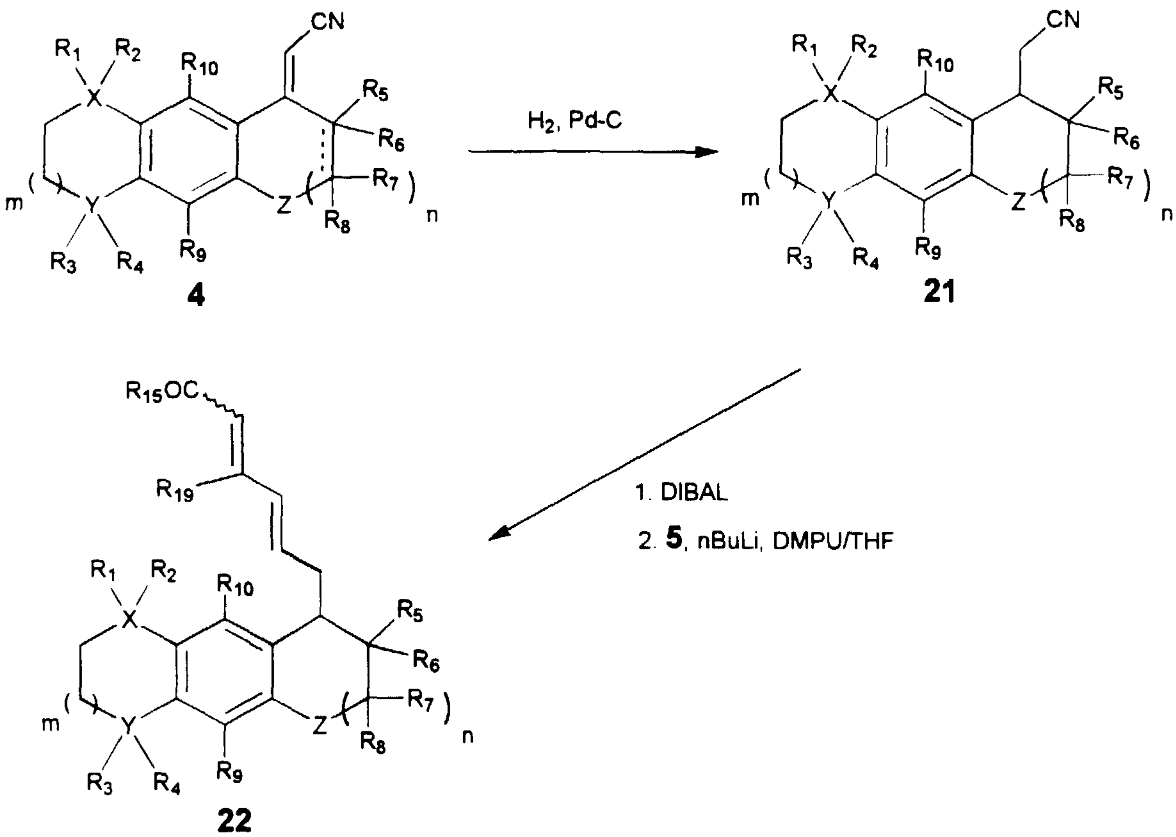


The tricyclic derivatives of structure **22** can be prepared in accordance with reaction Scheme VI. The previously described unsaturated nitriles **4** (see process for reaction Scheme I) are reduced by catalytic hydrogenation over 10% palladium-on-carbon in ethyl acetate to provide the saturated nitriles **21**. In accordance with the processes employed in reaction Scheme I, the nitriles can be transformed into the tricyclic derivatives of general structure **22**.

## Scheme VII: Synthesis of Compounds of Structure (II):

### Scheme VII.

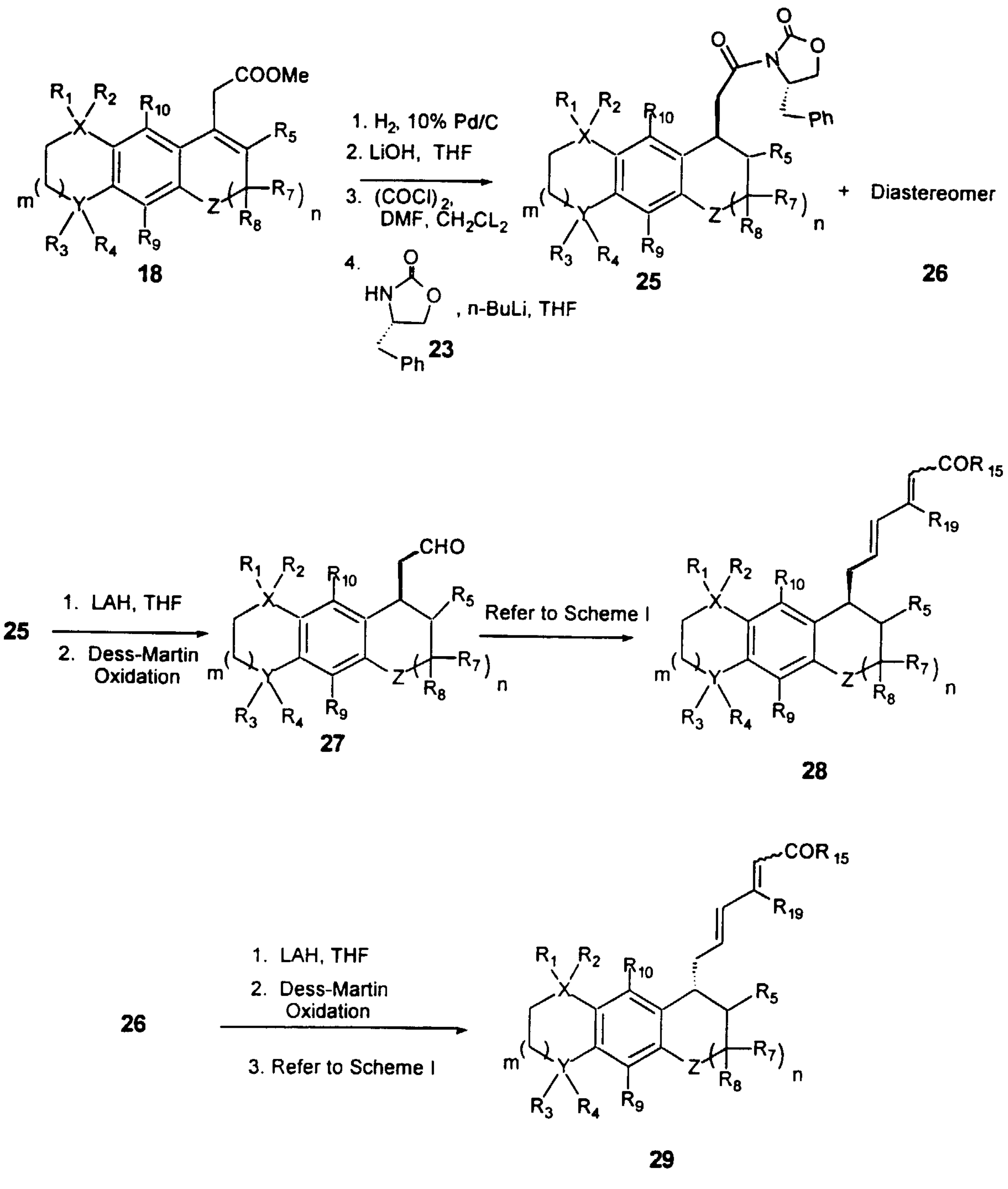


18 25 26 23 27 28 29

The chiral tricyclic derivatives of general structure **28** and **29** can be prepared in accordance with reaction Scheme VII. The previously described β,γ-unsaturated esters **18** (see reaction Scheme V) are reduced by catalytic hydrogenation over 10% palladium-on-carbon in ethyl acetate to provide the intermediate saturated esters which are converted to the diastereomeric acyl oxazolidinones of general structure **25** and **26** by coupling of the acid chloride derivative with (S)4-benzyl-2-oxazolidinone. The mixture of amide diastereomers is separated by silica gel chromatography and each is separately reduced with LAH at reduced temperatures in THF, followed by Dess-Martin oxidation

in methylene chloride at ambient temperature to provide the diastereomerically pure tricyclic aldehyde, for example tricyclic aldehydes of general structure **27**. In accordance with the processes employed in reaction Scheme I, the aldehydes are transformed into the tricyclic derivatives of general structure **28** and **29**.

## Scheme VIII: Synthesis of Compounds of Structure (II):

### Scheme VIII.

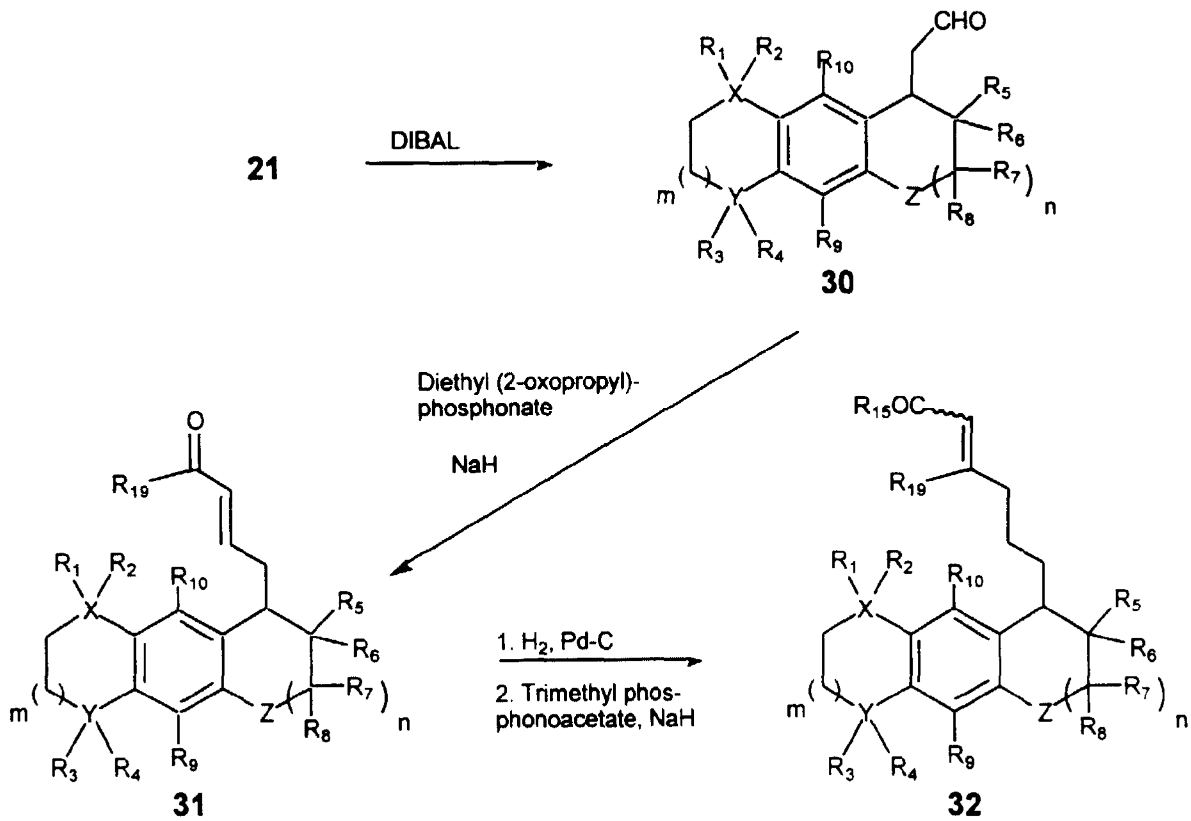


21 30 31 32

The tricyclic derivatives of general structure **32** can be prepared in accordance with reaction Scheme VIII. The previously described saturated nitriles **21** (see Scheme VI) are reduced with DIBAL in methylene chloride at -78°C to provide aldehydes of general structure **30**. The tricyclic aldehydes can be condensed with the sodium salt of diethyl (2-oxopropyl)-phosphonate in THF at reduced temperatures in a Horner-Wadsworth-Emmons olefination reaction to provide the α,β-unsaturated ketones **31**. The enones **31** can be reduced by catalytic hydrogenation over 10% palladium-on-carbon in ethyl acetate to provide the intermediate saturated ketones which are condensed with the sodium salt of trimethyl phosphonoacetate in THF at reduced temperatures in a Horner-Wadsworth-Emmons olefination reaction to provide β,γ-unsaturated esters of general structure **32**. The acids and salts derived from **32** are readily obtainable from the corresponding esters by the same processes as those employed in the preparation process of Scheme I for tricycles of general structure **6** and **7**.

## Scheme IX: Synthesis of Compounds of Structure (III):

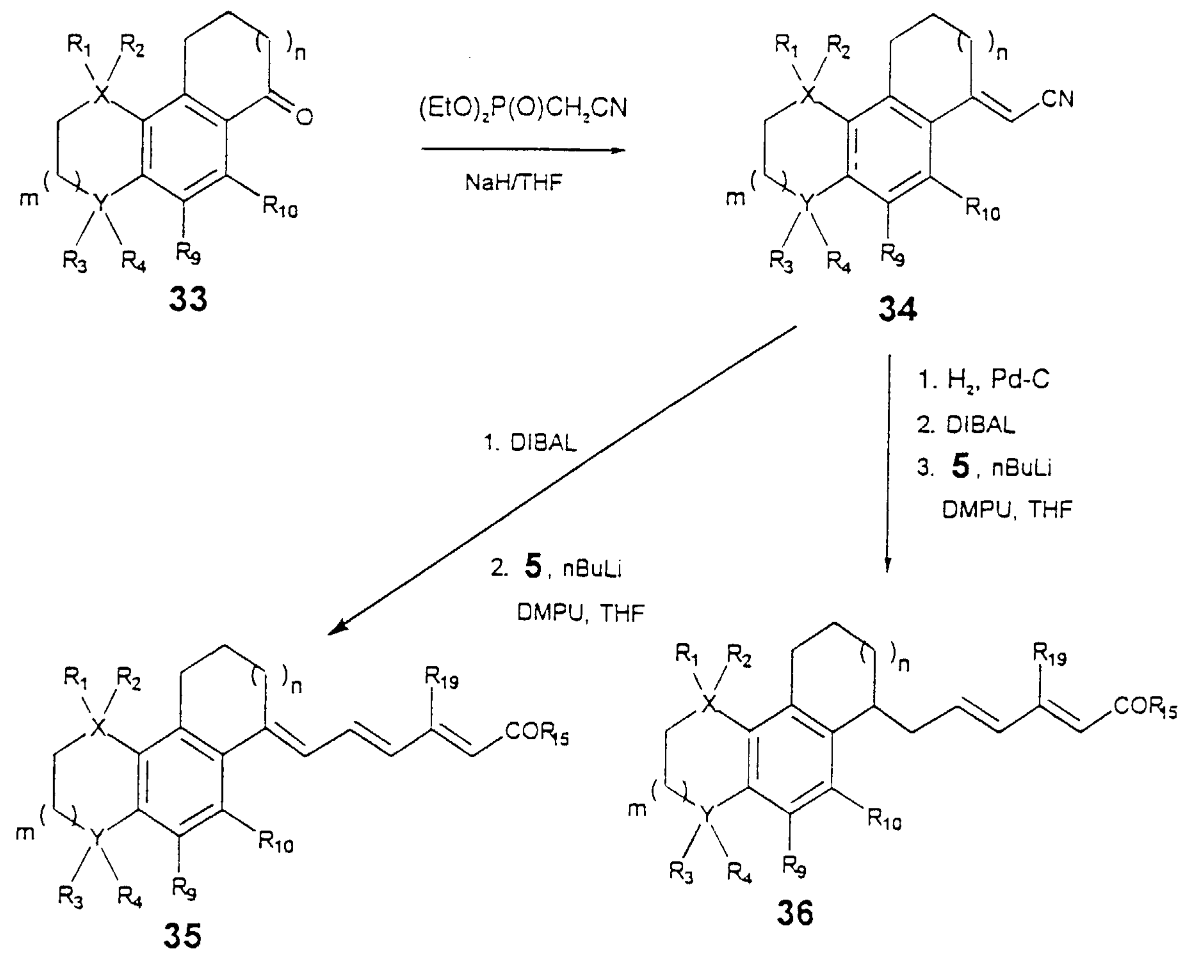


33 34 35 36

The tricyclic derivatives according to the present invention of general structures **35** and **36** can be prepared in accordance with reaction Scheme LX. The tricyclic trienes **35** can be prepared from the corresponding tricyclic ketones **33** in a manner analogous to that described in the preparation process of Scheme I for tricyclic derivatives of structures **6** and **7**. The tricyclic dienes of general structure **36** are prepared from the tricyclic ketones **33** in a manner analogous to that described in the preparation process of Scheme I and Scheme VI for tricyclic derivatives of general structure **22**.

**Scheme X: Synthesis of Compounds of Structure (II):**

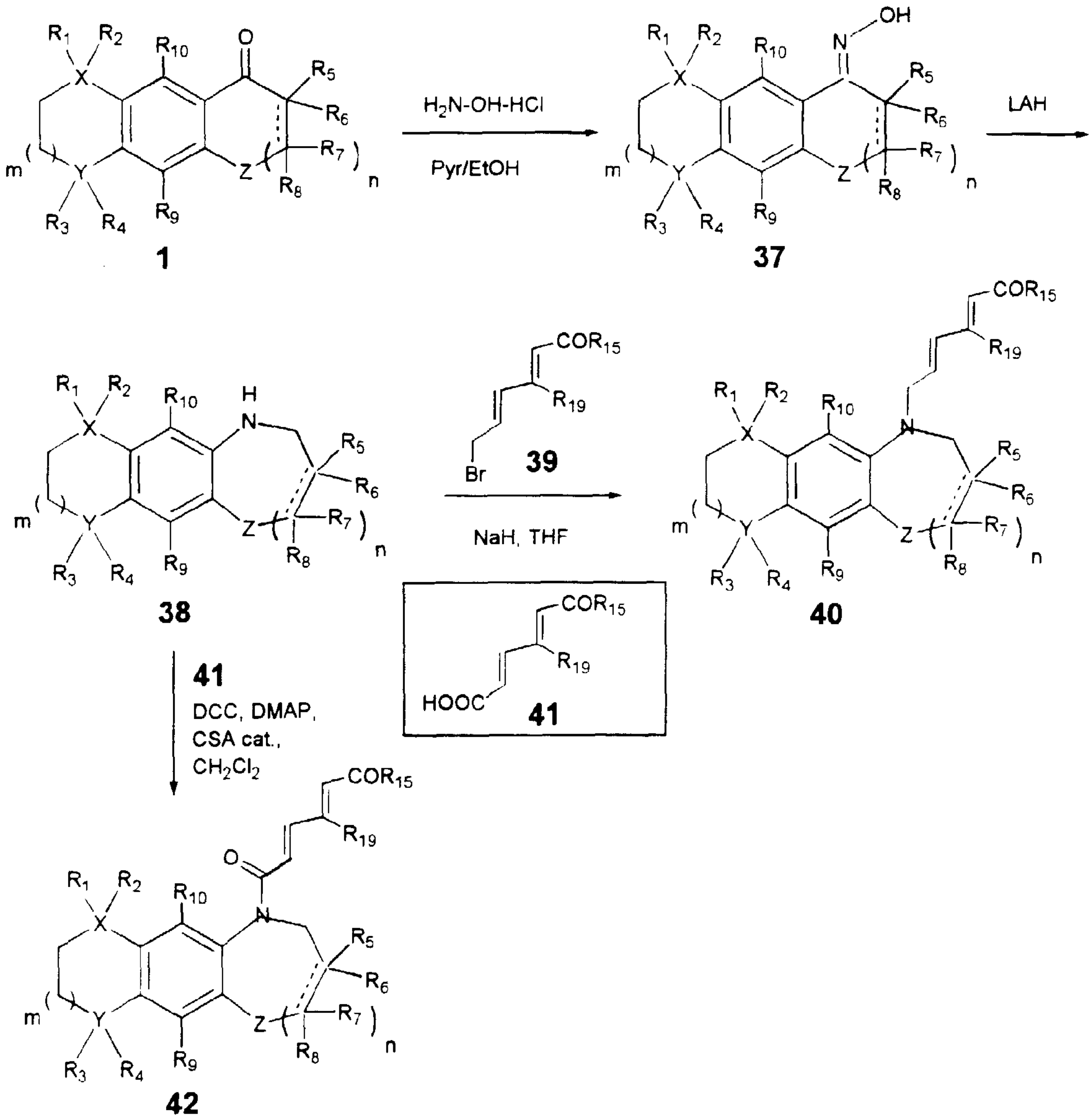


The tricyclic derivatives of general structures **40** and **42** can be prepared in accordance with reaction Scheme X. The previously described tricyclic ketones **1** (see preparation process for reaction Scheme I) are heated at reflux with hydroxylamine hydrochloride in pyridine and ethanol to provide oximes **37**. A Beckman rearrangement is effected with LAH in ethanol at 80°C to afford the corresponding tricyclic amines **38**, which are deprotonated with NaH at 0°C in THF and alkylated at ambient temperature with ethyl (2E,4E)-6-bromo-3-methylhexa-2,4-dienoate to give aminodienes of general structure **40**. Alternatively, the tricyclic amines **38** can be acylated by a DCC coupling in methylene chloride with (2E,4E)-3-methyl-5-carboxypenta-2,4-dienoate to give amides of general structure **42**. The acids and salts derived from the esters are readily obtainable by the same processes as those employed in the preparation process of Scheme I for tricycles of general structure **6** and **7**.

## Scheme XI: Synthesis of Compounds of Structure (II):

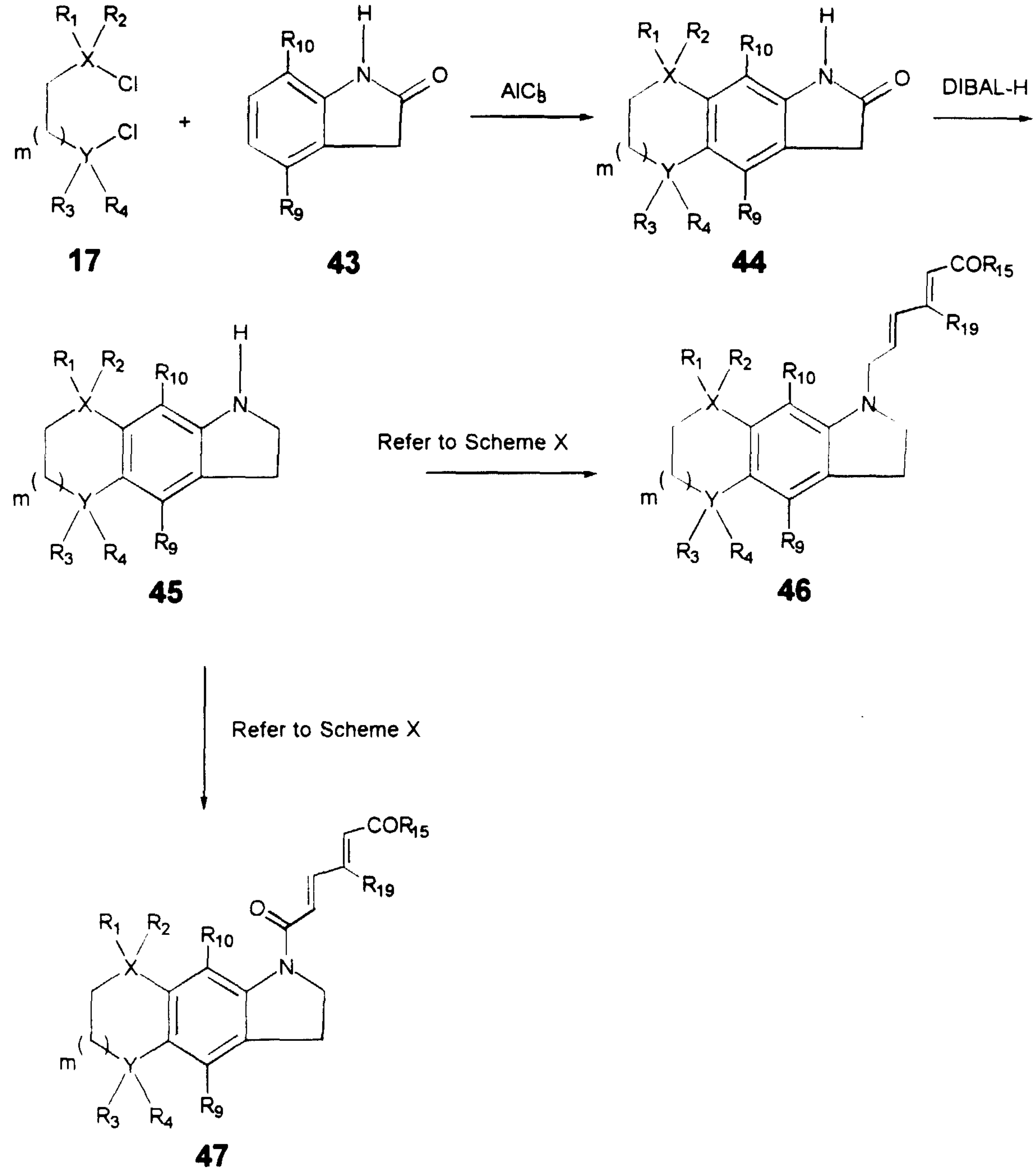


17 43 44 45 46 47

The tricyclic derivatives of general structure **46** and **47** can be prepared in accordance with reaction Scheme XI. The tricyclic amides **44** can be prepared from oxindole of general structure **43** and 2,5-dichloro-2,5-dialkylhexanes of general structure **17** by aluminum trichloride catalyzed Friedel-Crafts alkylation/cyclization in dichloromethane at ambient temperature. Amides of general structure **44** can be reduced with DIBAL in methylene chloride at 25°C to provide the corresponding amines **45**. The amine dienes **46** can be prepared from the amines **45** in a manner analogous to that described in the preparation process of Scheme X for tricyclic derivatives of general structure **40**. The desired tricyclic amides **47** are prepared from the corresponding tricyclic amines of **45** in a manner analogous to that described in the preparation process of Scheme X for tricyclic derivatives of general structure **42**. The acids and salts derived from **46** and **47** are readily obtainable from the corresponding esters by the same processes as those employed in the Scheme I for tricycles of structures **6** and **7**.

## Scheme XII: Synthesis of Compounds of Structure (III):

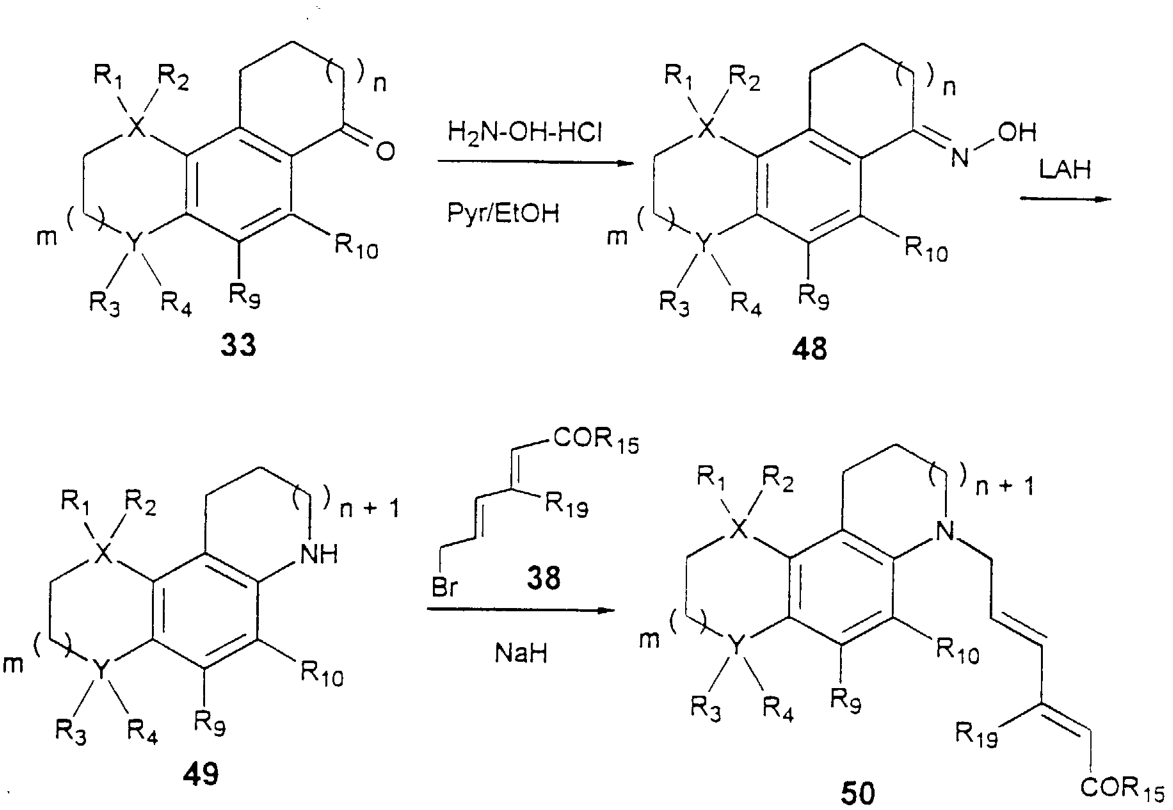


33 48 38 49 50

The tricyclic derivatives according to the present invention of general structure **50** can be prepared in accordance with reaction Scheme XII. The tricyclic ketones **33** are transformed to amines of general structure **49** via Beckman type rearrangement of the intermediate oximes **48**. Alkylation of the derived amines **49** with suitable alkyl halides of the type **38** provide the desired tricylcic derivatives of general structure **50** The acids and salts derived from **50** are readily obtain readily obtainable from the corresponding esters by the same processes as those employed in Scheme I for tricycles of structures **6** and **7**.

The structural formulas for Compounds of the present invention are given on the following pages.

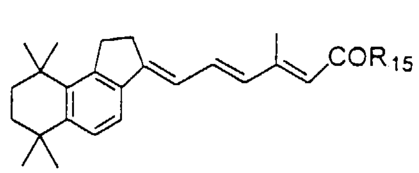

**135a:** $R_{15}$ = ethoxy
**135b:** $R_{15}$ = OH

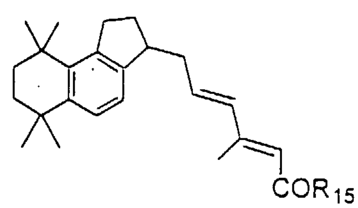

**136a:** $R_{15}$ = ethoxy
**136b:** $R_{15}$ = OH

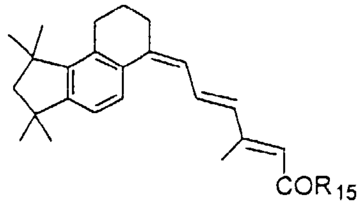

**137a:** $R_{15}$ = ethoxy
**137b:** $R_{15}$ = OH

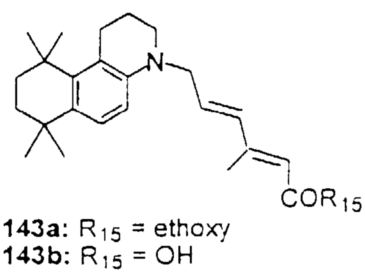

**143a:** $R_{15}$ = ethoxy
**143b:** $R_{15}$ = OH

In another aspect, the retinoid compounds of the present invention are combined in a mixture with a pharmaceutically acceptable carrier to provide pharmaceutical compositions useful for treating the biological conditions or disorders noted herein in mammalian, and more preferably, in human patients. The particular carrier employed in these pharmaceutical compositions may take a wide variety of forms depending upon the type of administration desired, e.g., intravenous, oral, topical, suppository or parenteral.

In preparing the compositions in oral liquid dosage forms (e.g., suspensions, elixirs and solutions), typical pharmaceutical media, such as water, glycols, oils, alcohols, flavoring agents, preservatives, coloring agents and the like can be employed. Similarly, when preparing oral solid dosage forms (e.g., powders, tablets and capsules), carriers such as starches, sugars, diluents, granulating agents, lubricants, binders, disintegrating agents and the like will be employed. Due to their ease of administration, tablets and capsules represent the most advantageous oral dosage form for the pharmaceutical compositions of the present invention.

For parenteral administration, the carrier will typically comprise sterile water, although other ingredients that aid in solubility or serve as preservatives, may also be included. Furthermore, injectable suspensions may also be prepared, in which case appropriate liquid carriers, suspending agents and the like will be employed.

For topical administration, the compounds of the present invention may be formulated using bland, moisturizing bases, such as ointments or creams. Examples of suitable ointment bases are petrolatum, petrolatum plus volatile silicones, lanolin, and water in oil emulsions such as Eucerin™ (Beiersdorf). Examples of suitable cream bases are Nivea™ Cream (Beiersdorf), cold cream (USP), Purpose Cream™ (Johnson & Johnson) hydrophilic ointment (USP), and Lubriderm™ (Warner-Lambert).

The pharmaceutical compositions and compounds of the present invention will generally be administered in the form of a dosage unit (e.g., tablet, capsule etc.) at from about 1 μg/kg of body weight to about 500 mg/kg of body weight, more preferably from about 10 μg/kg to about 250 mg/kg, and most preferably from about 20 μg/kg to about 100 mg/kg. As recognized by those skilled in the art, the particular quantity of pharmaceutical composition according to the present invention administered to a patient will depend upon a number of factors, including, without limitation, the biological activity desired, the condition of the patient, and tolerance for the drug.

The compounds of this invention also have utility when labeled and used in assays to determine the presence of RARs and RXRs. They are particularly useful due to their ability to selectively bind to members of the RAR and RXR subfamilies and can therefore be used to determine the presence of RAR and RXR isoforms in the presence of other retinoid receptors or related intracellular receptors.

Due to the selective specificity of the compounds of this invention for retinoid receptors, these compounds can also be used to purify samples of RARs and RXRs *in vitro.* Such purification can be carried out by mixing samples containing retinoid receptors with one of more of the compounds of the present invention, so that the compound (ligand) binds to the receptor, and then separating out the bound ligand/ receptor combination by separation techniques which are known to those of skill in the art. These techniques include column separation, filtration, centrifugation, tagging and physical separation, and antibody complexing, among others.

The compounds of the present invention also include racemate, individual stereoisomers and mixtures thereof. These isomers are then isolated by standard resolution techniques, including fractional crystallization and reverse phase and chiral column chromatography.

The compounds and pharmaceutical compositions of the present invention can advantageously be used in the treatment of the diseases and conditions described herein. In this regard, the compounds and compositions will prove particularly useful in the treatment of skin-related diseases and conditions, such as acne, psoriasis, and photo damage, cancerous and precancerous conditions, diseases of the eye, cardiovascular diseases, inflammatory and neurodegenerative diseases, diseases associated with human papilloma virus, improper pituitary function, modulation of apoptosis, diseases of the immune system, wound healing and restoration of hair growth.

Furthermore, the compounds and pharmaceutical compositions of the present invention possess a number of advantages over previously identified retinoid compounds. For example, the compounds are extremely potent activators of RARs and RXRs, preferably displaying 50% maximal activation of one or more of the retinoid receptors at a concentration of less than 100 nM, more preferably at a concentration of less than 50 nM, more preferably yet at a concentration of less than 20 nM, and most preferably at a concentration of less than 10 nM. Also, the RAR and RXR selective compounds of the present invention preferentially activate one subfamily of retinoid receptors at a level at least 2 times greater, preferably at least 5 times greater, more preferably at least 10 times greater, and most preferably at least 100 times greater than the other subfamily of retinoid receptors. In addition, the compounds of the present invention also are easier to synthesize, provide greater stability and bioavailability, and appear to be less teratogenic in comparison to all-*trans* retinoic acid and *9-cis* retinoic acid, known RAR and RXR active compounds, respectively.

The invention will be further illustrated by reference to the following non-limiting Examples.

## EXAMPLE 1

Preparation of compound **101a** according to Scheme I

**Ethyl (2E,4E)-3-methyl-6-[(Z)-3,4,5,6,7,8-hexahydro-5,5,8,8-tetramethyl-2H-anthracen-1-ylidene]hexa-2,4-dienoate** (structure **6**, where $R_1$, $R_2$, $R_3$, $R_4$, and $R_{19}$ are methyl, $R_5$, $R_6$, $R_7$, $R_8$, $R_9$, and $R_{10}$ are hydrogen, $R_{15}$ is ethoxy, X, Y and Z are carbon, m = n = 1)

To a solution of 1,2,3,4,6,7,8,9-octahydro-6,6,9,9-tetramethylanthracene (2.0 g, 8.3 mmol) [prepared by Friedel-Crafts alkylation/annulation of 1,2,3,4-tetrahydronaphthalene with 2,5-dichloro-2,3-dimethylhexane in the presence of aluminum trichloride at 0°C in dichloromethane] in $CH_2Cl_2$ (100 ml) and pyridine (15 ml) at 0°C was added $CrO_3$ (8.26 g, 82.6 mmol) in several portions. The reaction mixture was stirred at 0°C for 30 min, then allowed to warm up to room temperature and stirred for 10 h. The reaction mixture was poured over an ice-acid mixture (IN HCl, 100ml), extracted with $Et_2O$ (200 ml), dried ($MgSO_4$), concentrated, and purified by column chromatography (25% ether in hexane) to give 3,4,5,6,7,8-hexahydro-5,5,8,8-tetramethyl-2H-anthracen-1-one (740 mg, 35%): $^1$H NMR(400 MHz, $CDCl_3$) δ 8.01(s, 1H, ArH), 7.17(s, 1H, ArH), 2.90(t, J=6.5 Hz, 2H, $CH_2$, benzylic), 2.60(t, J=6.3 Hz, 2H, $CH_2$), 2.10(m, 2H, $CH_2$), 1.68(s, 4H, $2CH_2$), 1.30(s, 6H, $2CH_3$), 1.29(s, 6H, $2CH_3$).

To a solution of diethyl cyanomethylphosphonate (1.8 g, 10.4 mmol) in THF (4 ml) and DMPU (4 ml) at 0°C was added NaH (375 mg, 65% in oil, 10.4 mmol) in one portion. The resulting solution was warmed to room temperature for 30 min. To this solution was added 3,4,5,6,7,8-hexahydro-5,5,8,8-tetramethyl-2H-anthracen-1-one (1.08 g, 4.2 mmol) in THF (2 ml). The mixture was then refluxed at 80°C for 3 hr, cooled, quenched with saturated $NH_4Cl$ (20 ml), extracted with $Et_2O$ (100 ml), dried ($MgSO_4$), concentrated, and purified by column chromatography (10% ether in hexane) to afford *cis*-(1,2,3,4,6,7,8,9-octahydro-6,6,9,9-tetramethyl-2H-anthracen-1-ylidene)ethanitrile (100 mg, 10%) and *trans*-(1,2,3,4,6,7,8,9-octahydro-6,6,9,9-tetramethyl-2H-anthracen-1-ylidene)ethanitrile (660 mg, 66%). The cis-nitrile (structure **3**) had $^1$H NMR(400 MHz, $CDCl_3$) δ 8.31(s, 1H, ArH), 7.09(s, 1H, ArH), 5.19(s, 1H, olefinic), 2.80(t, J=6.5 Hz, 2H, $CH_2$, benzylic), 2.55(t, J=6.3 Hz, 2H, $CH_2$), 1.90(m, 2H, $CH_2$), 1.64(s, 4H, $2CH_2$), 1.26(s, 6H, $2CH_3$), 1.25(s, 6H, $2CH_3$). The *trans*-nitrile (structure **4**) had $^1$H NMR(400 MHz, $CDCl_3$) δ 7.46(s, 1H, ArH), 7.09(s, 1H, ArH), 5.68(s, 1H, CH, olefinic), 2.80(m, 4H, $2CH_2$), 1.90(m, 2H, $CH_2$), 1.65(s, 4H, $2CH_2$), 1.26(s, 6H, $2CH_3$), 1.25(s, 6H, $2CH_3$).

To a solution of the above Z-(3,4,5,6,7,8-hexahydro-5,5,8,8-tetramethyl-2H-anthracen-1-ylidene)ethanitrile (56 mg, 0.2 mmol) in $CH_2Cl_2$ (2 ml) at -78°C was added DIBAL (0.4 ml, 1M in $CH_2Cl_2$, 0.4 mmol). The mixture was stirred at that temperature for 10 min, then quenched with saturated potassium sodium tartrate (10 ml) at -78°C, warmed to room temperature, extracted with EtOAc (50 ml), dried ($MgSO_4$), and concentrated to give essentially pure aldehyde, which was employed for the next reaction without further purification.

A solution of diethyl 3-ethoxycarbonyl-2-methylprop-2-enylphosphonate (86 mg, 0.33 mmol) in THF (1 ml) and DMPU (1 ml) at 0°C was treated with nBuLi (0.13 ml, 2.5 M in hexane, 0.33 mM), then warmed to room temperature for 30 min. The solution was cooled to -78°C, and the above aldehyde (32 mg, 0.11 mmol) in THF (1 ml) was slowly

added. Subsequently, the reaction mixture was allowed to warm to room temperature for 30 min, quenched with a saturated solution of $NH_4Cl$ (5 ml), extracted with Et20 (50 ml), dried (MgS04), concentrated, and purified by column chromatography (10% ether in hexane) to give the title ester (**101a**) (39 mg, 85%): Rf= 0 48(10 % ether in hexane); $^1H$ NMR(400 MHz, $CDCl_3$) δ 7.33(s, 1H, ArH), 7.19(dd, J=15.2, 11.2 Hz, 1H, olefinic), 7.07(s, 1H, ArH), 6.32(d, J=15.2 Hz, 1H, olefinic), 6.14(d, J=11.2 Hz, 1H, olefinic), 4.15(q, 2H, $OCH_2$), 2.79(t, J=6.5 Hz, 2H, $CH_2$), 2.50(t, J=5.8 Hz, 2H, $CH_2$), 2.28(s, 3H, $CH_3$), 1.91(m, 2H, $CH_2$), 1.68(s, 4H, $2CH_2$), 1.29(s, 6H, $2CH_3$), 1.27(s, 6H, $2CH_3$), 1.24(t, J=6.8 Hz, 3H, $CH_3$).

## EXAMPLE 2

Preparation of compound **101b** according to Scheme I

**(2E,4E)-3-Methyl-6-[(Z)-3,4,5,6,7,8-hexahydro-5,5,8,8-tetramethyl-2H-anthracen-1-ylidene]hexa-2,4-dienoic acid** (structure **6**, where $R_1$, $R_2$, $R_3$, $R_4$, and $R_{19}$ are methyl, $R_5$, $R_6$, $R_7$, $R_8$, $R_9$, and R10 are hydrogen, $R_{15}$ is hydroxy, X, Y, and Z are carbon, m = 1, and n = 1)

To a solution of ester **101a** (39 mg, 0.1 mmol) in MeOH (1 ml) and $H_2O$ (1 ml) was added KOH (100 mg, 2.5 mmol) at 25°C. The reaction mixture was heated at 80°C for 3 hr, cooled, acidified (1.1 ml, 2 4 N HCl), extracted with Et20 (40 ml), dried (MgS04), concentrated, and purified using column chromatography (50% ether in hexane) to obtain the title compound **101b** (35 mg, 95%): $R_f$= 0.32(50 % ether in hexane); mp 225 - 227°C; $^1H$ NMR(400 A4Fb, $CDCl_3$) 7.32(s, 1H, ArH), 7.24(dd, J=15.2, 11.2 Hz, $^1H$, olefinic), 7.07(s, 1H, ArH), 6.34(d, J=15.2 Hz, 1H, olefinic), 6.16(d, J=11.2 Hz, $^1H$, olefinic), 5.80(s, 1H, olefinic), 2.79(t, J=6.4 Hz, 2H, $CH_2$, benzylic), 2.51(t, J=5.8 Hz, 2H, $CH_2$), 2.34(s, 3H, $CH_3$), 1.92(m, 2H, $CH_2$), 1.68(s, 4H, $2CH_2$), 1.29(s, 6H, $2CH_3$), 1.27(s, 6H, $2CH_3$).

## EXAMPLE 3

Preparation of compound **102a** according to Scheme I

**Ethyl (2E,4E)-3-methyl-6-[(Z)-2,3,5,6,7,8-hexahydro-5,5,8,8-tetramethylcyclopenta[b]napthalen-1-ylidene] hexa-2,4-dienoate** (structure **6**, where $R_1$, $R_2$, $R_3$, $R_4$, and $R_{19}$ are methyl, $R_5$, $R_6$, $R_7$, $R_8$, $R_9$, and $R_{10}$ are hydrogen, $R_{15}$ is ethoxy, X, Y, and Z are carbon, m = 1, and n = 0).

The title compound was prepared in a manner similar to that of compound **101a** except that 2,3,5,6,7, 8-hexahydro-5,5,8,8-tetramethylcyclopenta[b]naphthalen 1-one [US patent 2,815,382 (1957)] was used as the starting ketone (structure **1**) in Example 1. Compound **102a** had $R_f$= 0.56(10 % ether in hexane); $^1H$ NMR(400 MHz, $CDCl_3$) δ 7.68(s, 1H, ArH), 7.45(dd, J=15.0, 11.6 Hz, 1H, olefinic), 7.24(s, 1H, ArH), 6.25( d, J=15.0 Hz, 1H, olefinic), 6.22(d, J=11.6 Hz, 1H, olefinic), 5.75(s, 1H, olefinic), 4.15(q, 2H, $OCH_2$), 2.88(bm, 2H, $CH_2$, benzylic), 2.79(bm, 2H, $CH_2$, allylic), 2.48(s, 3H, $CH_3$), 1.68(s, 4H, $2CH_2$), 1.31(s, 26H, $CH_3$), 1.29(m, 9H, $3CH_3$).

## EXAMPLE 5

Preparation of compound **103a** according to Scheme I

**Ethyl (2E,4E)-3-methyl-6-[(Z)-1,2,3,6,7,8-hexahydro-1,1,3,3-tetramethylcycopenta[b]naphthalen-5-ylidene] hexa-2,4-dienoate** (structure **6**, where $R_1$, $R_2$, $R_3$, $R_4$ and $R_{19}$ are methyl, $R_5$, $R_6$, $R_7$, $R_8$, $R_9$, and $R_{10}$ are hydrogen, $R_{15}$ is ethoxy, X, Y, and Z are carbon, m=0 and n=1).

The title compound was prepared from 1,2,3,6,7,8-hexahydro-1,1,3,3-tetramethylcyclopenta[b]naphthalen-5-one. The preparation of this naphthalen-5-one was achieved as follows. 3,3-Dimethylcrotyl chloride was treated with 1,2,3,4-tetrahydronaphthalene in the presence of aluminum trichloride to give the corresponding 1,2,3,5,6,7,8-heptahdyro-1,1-dimethyl-3-oxo-cyclopenta[b]naphthalene, which was then geminally dimethylated with dimethyl zinc in the presence of titanium tetrachloride to afford 1,2,3,5,6,7,8-heptahdyro-1,1,3,3 -tetramethyl-cydopenta[b]naphthalene. The naphthalene derivative was then oxidized using $CrO_3$ in acetic acid in a manner similar to that described in Example 1. The title compound **103a** had $R_f$=0.85 (10% ether in hexane); $^1H$ NMR (400 MHz, $CDCl_3$) δ 7.20 (dd, J=15.2, 11.2 Hz, 1H, olefinic), 7.15 (s, 1H, ArH), 6.91 (s, 1H, ArH), 6.32 (d, J=15.2 Hz, 1H, olefinic), 6.19 (s, J=11.2 Hz, 1H, olefinic), 5.77 (s, 1H, olefinic), 4.16 (q, 2H, $OCH_2$), 2.83 (t, J=6.5 Hz, 2H, $CH_2$ benzylic), 2.53 (t, J=6.1 Hz, 2H, $CH_2$ allylic), 2.28 (s, 3H, $CH_3$), 1.91 (m, 2H, $CH_2$, methylene), 1.90 (s, 3H, $CH_2$, methylene), 1.43 (s, 6H, $2CH_3$), 1.32 (s, 6H, $2CH_3$), 1.27 (t, J=6.7 Hz, 3H, $CH_3$).

**EXAMPLE 14**

Preparation of compound **109a** according to Scheme III

**Ethyl (2E,4E)-3-methyl-6-[(E)-3,4,5,6,7,8-hexahydro-5,5,8,8-tetramethyl-2H-anthracen-1-ylidene]hexa-2,4-dienoate** (structure **7**, where $R_1$, $R_2$, $R_3$, $R_4$, and $R_{19}$ are methyl; $R_5$, $R_6$, $R_7$, $R_8$, $R_9$, $R_{10}$ are hydrogen; $R_{15}$ is ethoxy; X, Y, and Z are carbon; m = n = 1).

To a solution of 3,4,5,6,7,8-hexahydro-5,5,8,8-tetramethyl-2H-anthracen-1-one (512 mg, 2 mmol, from Example 1) in MeOH(10 ml) was added NaBH4 (76 mg, 2 mmol) at 0°C. The reaction mixture was stirred at that temperature for 30 min, then quenched with sat, aqueous $NH_4Cl$ (5 ml), extracted with ether (50 ml), dried ($MgSO_4$), and concentrated under reduced pressure to give the corresponding tricyclic alcohol, which was used without further purification. To the above alcohol (516 mg, 2 mmol) in MeOH (5 ml) was added $Ph_3P$-HBr (686 mg, 2 mmol) at 25°C. The mixture was heated at 85°C for 5 h. Removal of the solvent, followed by addition of hexane (100 ml) gave a white solid, which was then filtered to give pure 3,4,5,6,7,8-hexahydro-5,5,8,8-tetramethyl-2H-anthracen-1-triphenylphosphonium bromide (697 mg, 60%).

To a solution of the above phosphonium salt (581 mg, 1 mmol) in THF (8 ml) was added nBuLi (0.4 ml, 2.5 M, 1 mM) at 0°C and the resulting dark-red solution was stirred at that temperature for 30 min to afford the ylide. To this freshly prepared ylide was added ethyl (2E,4E)-3-methyl-1-formylpenta-2,4-dienoate. [The ethyl dienoate was prepared by the condensation of ethyl-3-methyl-4-oxocrotonate and (triphenylphosphoranylidene)acetaldehyde in benzene at 85°C for 3 h using benzoic acid as a catalyst (168 mg, 1 mmol) in THE(5 ml) at 0°C, and the resulting mixture was stirred at this temperature for 30 min.] The reaction mixture was quenched with $NH_4Cl$ (10 ml), extracted with ether (50 ml), dried($MgSO_4$), concentrated, and purified by chromatography (10% ether in hexane) to give the pure title compound **109a** (372 mg, 95%): $R_f$=0.52 (10% ether in hexane); $^1H$ NMR(400 MHz, $CDCl_3$) δ 7.58 (s, 1H, ArH), 7.04 (dd, J=11.2, 15.0 Hz, 1H, olefinic), 7.02 (s, 1H, ArH), 6.69 (d, J=11.2 Hz, 1H, olefinic), 6.39 (d, J=15.0 Hz, 1H, olefinic), 5.79 (s, 1H, olefinic), 4.16 (q, 2H, $OCH_2$), 2.75 (t, J=6.1 Hz, 2H, $CH_2$, benzylic), 2.68 (t, J=5.9 Hz, 2H, $CH_2$, allylic), 2.36 (s, 3H, $CH_3$), 1.85 (m, 2H, $CH_2$), 1.65 (s, 4H, $2CH_2$), 1.31 is, 6H, $2CH_3$), 1.25 (m, 9H, $3CH_3$).

**EXAMPLE 37**

Preparation of compound **124a** according to Scheme VI

**Ethyl (2E,4E)-3-methyl-6-(1,2,3,4,5,6,7,8-octahydro-5,5,8,8-tetramethylanthracen-1-yl)-hexa-2,4-dienoate** (structure **22**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is ethoxy; X, Y, and Z are carbon; m = n=1).

To a solution of [(E)-3,4,5,6,7,8,9-hexahydro-5,5,8,8-tetramethyl-2H-anthracen-1-ylidene]ethanitrile (56 mg, 0.2 mmol from Example 1) in EtOAc (5 ml) was added 5 mg of 10% Pd-C catalyst, and the reaction mixture was treated with hydrogen gas at 1 atm for 3 h. Removal of the catalyst by filtration, and concentration of organic solvent gave essentially pure saturated nitrile which was used for the next reaction without further purification. The saturated nitrile was then sequentially subjected to DIBAL reduction and Wittig coupling reaction using conditions similar to those described in Example 1 to afford, after column chromatography, the title compound **124a**: $R_f$=0.55(10 % ether in hexane); $^1H$ NMR(400 MHz, $CDCl_3$) δ 7.09 (s, 1H, ArH), 6.98 (s, 1H, ArH), 6.20 (m, 1H, olefinic), 6.12 (d, J=15.7 Hz, 1H, olefinic), 5 70 (s, 1H, olefinic), 4.13 (q, 2H, $OCH_2$), 2.85 (m, 1H, CH), 2.79 (br t, 2H, $CH_2$, benzylic), 2.60 (m, 1H, CH, allylic), 2.37 (m, $^1H$, CH, allylic), 2.30 (s, 3H, $CH_3$), 1.80 (m, 2H, $CH_2$, methylene), 1.65 (m, 2H, $CH_2$, methylene), 1.63 (s, 6H, $2CH_2$), 1.27 (s, 6H, $2CH_3$), 1.26 (s, 6H, $2CH_3$), 1.26 (t, J=6.8 Hz, 3H, $CH_3$).

**EXAMPLE 51**

Preparation of compound **135a** according to Scheme IX

**Ethyl (2E,4E)-3-methyl-6-[(E)-2,3,6,7,8,9-hexahydro-6,6,9,9-tetramethyl-1H-cyclopenta[a]naphthalen-3-ylidene]hexa-2,4-dienoate** (structure **35**, where $R_1$, $R_2$, $R_3$, $R_4$, and $R_{19}$ are methyl; $R_9$ and $R_{10}$ are hydrogen; $R_{15}$ is ethoxy; X and Y are carbon; m = 1, and n = 0).

The title compound was prepared from 2,3,6,7,8,9-hexahydro-6,6,9,9-tetramethyl-1H-cyclopenta[*a*]naphthalen-3-one [a by-product isolated from the preparation of 2,3,5,6,7,8-hexahydro-5,5,8,8-tetramethyl-cyclopenta[*b*]naphthalen-1-one in Example 3] in a manner similar to that described for compound **51a** using the procedure described in

Example 1. Compound **135a** had $^1H$ NMR (400 MHz, $CDCl_3$) δ 7.36 (d, J=8.27 Hz, $^1H$, ArH), 7.26 (d, J=8.27 Hz, 1H, ArH), 6.90 (dd, J=11.2, 15.1 Hz, 1H, olefinic), 6.59 (d, J=11.2 Hz, 1H, olefinic), 6.31 (d, J=15.1 Hz, 1H, olefinic), 5.78 (s, CH, olefinic), 4.18 (q, J=7.02 Hz, 2H, $OCH_2$), 3.23 (br m, 2H, benzylic), 2.92 (br m, 2H, allylic), 2.37 (s, 3H, $CH_3$), 1.69 (br s, 4H, $2CH_2$), 1.36 (s. 6H, $2CH_3$), 1.32 (s, 6H, $2CH_3$), 1.31 (t. J=7.0 Hz. 3H, $CH_3$).

**EXAMPLE 52**

Preparation of compound **135b** according to Scheme IX

**(2E,4E)-3-Methyl-6-[(E)-2,3,6,7,8,9-hexahydro-6,6,9,9-tetramethyl-1H-cyclopenta[a]naphthalen-3-ylidene] hexa-2,4-dienoic acid** (structure **35**, where $R_1$, $R_2$, $R_3$, $R_4$, and $R_{19}$ are methyl; $R_9$ and $R_{10}$ are hydrogen; $R_{15}$ is hydroxy; X and Y are carbon; m = 1, and n = 0).

The title compound was prepared from compound **135a** using the standard hydrolysis condition described in Example 2. Acid **135b** had mp 195-197°C; $^1H$ NMR (400 MHz, $CDCl_3$) δ 7.30 (d, J=8.2 Hz, 1H, ArH), 7.21 (d, J=8.2 Hz, 1H, ArH), 6.88 (dd, J=11.3, 15.1 Hz, 1H, olefinic), 6.54 (d, J=11.3 Hz, 1H, olefinic), 6.27 (d, J=15.1 Hz, 1H, olefinic), 5.74 (s, 1H, olefinic), 3.17 (br m, 2H, $CH_2$, benzylic), 2.86 (m, 2H, $CH_2$, allylic), 2.32 (s, 3H, $CH_3$), 1.62 (br s, 4H, $2CH_2$), 1.28 (s, 6H, $2CH_3$), 1.24 (s, 6H, $2CH_3$).

**EXAMPLE 53**

Preparation of compound **136a** according to Scheme IX

**Ethyl (2E,4E)-3-methyl-6-(2,3,6,7,8,9-hexahydro-6,6,9,9-tetramethyl-1H-cyclopenta[a]naphthalen-3-yl]hexa-2,4-dienoate** (structure **36**, where $R_1$, $R_2$, $R_3$, $R_4$, and $R_{19}$ are methyl; $R_9$ and $R_{10}$ are hydrogen; $R_{15}$ is ethoxy; X and Y are carbon; m = 1, and n = 0)..

The title compound was prepared from (2,3,6,7,8,9-hexahydro-6,6,9,9-tetramethyl-1H-cyclopenta[a]naphthalen-3-ylidene)ethanitrile, which was prepared from the condensation of 2,3,6,7,8,9-hexahydro-6,6,9,9-tetramethyl 1H-cyclopenta[a]naphthalen-3-one (Example 51) with cyanomethylphosphonate using NaH as a base in a manner similar to that described for compound **124a** as detailed in Example 37. Compound **136a** had $^1H$ NMR(400 MHz, $CDCl_3$) δ 7.20 (d, J=8.0 Hz, 1H, ArH), 7.03 (d, J=8.0 Hz, 1H, ArH), 6.18 (m, 2H, 2 x olefinic), 5.71 (s, 1H, olefinic), 4.17 (q, J=7.6 Hz, 2H, $OCH_2$), 3.09 (m, 2H, $CH_2$, benzylic), 2.97 (m, 1H, benzylic), 2.67 (m, 1H, allylic), 2.86 (s, 3H, $CH_3$), 2.19 (m, 1H, allylic), 1.66 (m, 6H, $3CH_2$), 1.34 (s, 6H, $2CH_3$), 1.33 (s, 6H, $2CH_3$), 1.24 (t, J=7.0 Hz, 3H, $CH_3$).

**EXAMPLE 54**

Preparation of compound **136b** according to Scheme IX

**(2E,4E)-3-Methyl-6-(2,3,6,7,8,9-hexahydro-6,6,9,9-tetramethyl-1H-cyclopenta[a]naphthalen-3-yl)hexa-2,4-dienoic acid** (structure **36**, where $R_1$, $R_2$, $R_3$, $R_4$, and $R_{19}$ are methyl; $R_9$ and $R_{10}$ are hydrogen; $R_{15}$ is hydroxy; X and Y are carbon; m = 1, and n = 0)

The title compound was prepared by hydrolysis of compound **136a** using the standard hydrolysis conditions described in Example 2. Compound **136b** had mp 208-2l0°C; $^1H$ NMR (400 MHz, $CDCl_3$) δ 7.22 (d, J=8.0 Hz, 1H, ArH), 7.03 (d, J=8.0 Hz, 1H, ArH), 6.22 (m, 2H, 2 x olefinic), 5.75 (s, 1H, olefinic), 3.10 (m, 2H, benzylic), 2.95 (m, 1H, benzylic), 2.70 (m, 1H, allylic), 2.30 (s, 3H, $CH_3$), 2.20 (m, 1H, allylic), 1.66 (m, 6H, $3CH_2$), 1.32 (s, 6H, $2CH_3$), 1.28 (s, 6H, $2CH_3$).

**EXAMPLE 55**

Preparation of compound **137a** according to Scheme IX

**Ethyl (2E,4E)-3-methyl-6-[(Z)-1,2,3,4,7,8,9-heptahydro-7,7,9,9-tetramethylcyclopenta[f]naphthalen-4-ylidene] hexa-2,4-dienoate** (structure **35**, where $R_1$, $R_2$, $R_3$, $R_4$, and $R_{19}$ are methyl; $R_9$ and $R_{10}$ are hydrogen; $R_{15}$ is ethoxy; X and Y are carbon; m = 0, and n = 1)

The title compound was prepared from 1,2,3,4,7,8,9-heptahydro-7,7,9,9-tetramethyl-cyclopenta[*f*]naphthalen-4-one (isolated as a by-product for the preparation of 1,2,3,6,7,8-hexahydro-1,1,3,3-tetramethyl-cyclopenta[b] naphthalen-5-one in Example 5) using the procedure described in example 51. Compound **137a** had $R_f$=0.50 (15% ether in hexane); $^1H$ NMR (400 MHz, $CDCl_3$) δ: 7.25 (d, J=7.9 Hz, 1H, ArH), 7.15 (dd, J=15.2, 11.4 Hz, 1H, olefinic), 7.00 (d, J=7.9 Hz, 1H, ArH), 6.30 (d, J=15.2 Hz, 1H, olefinic), 6.14 (d, J=11.4 Hz, 1H, olefinic), 5.76 (s, 1H, olefinic), 4.16 (q, 2H, $OCH_2$), 2.88 (t, J=6.4 Hz, 2H, benzylic), 2.51 (t, J=6.5 Hz, 2H, allylic), 2.29 (s, 3H, $CH_3$), 1.91 (m, 4H, $2CH_2$), 1.42 (s, 6H, $2CH_3$), 1.29 (m, 9H, $3CH_3$).

## EXAMPLE 56

Preparation of compound **137b** according to Scheme IX

**(2E,4E)-3-Methyl-6-[(Z)-1,2,3,4,7,8,9-heptahydro-7,7,9,9-tetramethylcyclopenta[f]naphthalen-4-ylidene]hexa-2,4-dienoic acid** (structure **35**, where $R_1$, $R_2$, $R_3$, $R_4$, and $R_{19}$ are methyl; $R_9$ and $R_{10}$ are hydrogen; $R_{15}$ is hydroxy; X and Y are carbon; m = 0, and n = 1)

The title compound was prepared from compound **137a** using the standard hydrolysis conditions described in Example 2. Compound **137b** had $R_f$=0.45 (50% ether in hexane), mp 176-178°C; $^1H$ NMR (400 MHz, $CDCl_3$) δ 7.24 (d, J=7.8 Hz, 1H, ArH), 7.19 (dd, J=15.2, 11.3 Hz, 1H, olefinic), 7.00 ( d, J=7.9 Hz, 1H, ArH), 6.33 (d, J=15.2 Hz, 1H, olefinic), 6.16 (d, J=11.3 Hz, 1H, olefinic), 5.79 (s, 1H, olefinic), 2.88 (t, J=6.4 Hz, 2H, benzylic), 2.53 (t, J=6.4 Hz, 2H, allylic), 2.30 (s, 3H, $CH_3$), 1.93 (m, 4H, $2CH_2$), 1.43 (s, 6H, $2CH_3$), 1.31 (m, 6H, $2CH_3$).

## EXAMPLE 57

Preparation of compound **138a** according to Scheme X

**Ethyl (2E,4E)-3-methyl-6-(7,7,10,10-tetramethyl-2,3,4,5,7,8,9,10-octahydronaphtho[2,3-6]-azepinyl)hexa-2,4-dienoate** (structure **40**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is ethoxy; X, Y, and Z are carbon; m = n = 1).

To a solution of 3,4,5,6,7,8-hexahydro-6,6,9,9-tetramethyl-2H-anthracen-1-one (256 mg, 1 mmol, from Example 1) in EtOH (10 ml) and pyridine (3 drops) was added $H_2NOH$-HCl(140 mg, 2 mmol) at 25°C, and resulting mixture was heated at reflux for 5 h. The mixture was cooled to room temperature and the filtered through filter paper and the solid was washed with hexane (50 ml). The solid was dried under vacuum to give pure *trans*-(3,4,6,7,8-hexahydro-6,6,9,9-tetramethyl-2H-anthracene-1-yl)-oxime (219 mg, 84%): $^1H$ NMR(400 MHz, $CDCl_3$) δ 7.85 (s, 1H, ArH), 7.05 (s, 1H, ArH), 2.76 (t, J=6.1 Hz, 2H, benzylic), 2.69 (t, J=6.0 Hz, 2H, $CH_2$), 1.85 (m, 2H, $CH_2$), 1.65 (s, 4H, $2CH_2$), 1.32 (s, 6H, $2CH_3$), 1.30 (s, 6H, $2CH_3$).

The above oxime (271 mg, 1 mmol) in THF(10 ml) was treated with $LiAlH_4$ (3 ml, 1 M, 3 mM) at room temperature, and the resulting mixture was heated at reflux (80°C) for 8 h. The reaction mixture was cooled to 0°C, quenched with sodium potassium tartrate (20 ml), extracted with EtOAc(100 ml), dried over $MgSO_4$ and concentrated under reduced pressure. The crude product was chromatographed (20% ether in hexane) to afford pure 5,6,7,8-tetrahydro-5,5,8,8-tetramethyl-(2,3)-naphthyl-[*b*]azepine (218 mg, 85%): $^1H$ NMR(400 MHz, $CDCl_3$) 6 6.95 (s, 1H, ArH), 6.62 (s, 1H, ArH), 3.01 (t, J=6.2 Hz, 2H, benzylic), 2.69 (br m, 2H, $CH_2$), 1.73 (m, 2H, $CH_2$), 1.62 (m, 2H, $CH_2$), 1.61 (s, 4H, $2CH_2$), 1.25 (s, 12H, $4CH_3$).

To a solution of the above azepine (257 mg, 1 mmol) in THF (5 ml) was added NaH (80 mg, 60 % in oil, 2 mmol) at 0°C, and the resulting mixture was stirred at 0°C for 30 min. To this solution was added ethyl (2E,4E)-3-methyl-6-bromohexa-2,4-dienoate (233 mg, 1 mmol) [prepared by $NaBH_4$ reduction of ethyl (2E,4E)-(3-methyl-5-formyl)penta-2,4-dienoate in Example 14, followed by bromination using PBr3 in ether at 0°C to afford the necessary bromo compound] in THE (5 ml) at room temperature, and the resulting mixture was stirred for 8 h. Standard work-up procedure as described in Example 1, followed by chromatographic purification (10% ether in hexane) gave the title ester (294 mg, 72%): $R_f$=0.78(20 % ether in hexane); $^1H$ NMR(400 MHz, $CDCl_3$) δ 6.97 (s, 1H, ArH), 6.75 (s, 1H, ArH), 6.32 (d, J=15.8 Hz, 1H, olefinic), 6.22 (dt, J=15.8, 5.6 Hz, 1H, olefinic), 5.72 (s, 1H, olefinic), 4.15 (q, J=7.0 Hz, 2H, $OCH_2$), 3.85 (br d, J=5.6 Hz, 2H, allylic), 2.86 (br t, 2H, $NCH_2$), 2.73 (m, 2H, benzylic), 2.25 (s, 3H, $CH_3$), 1.66 (m, 4H, $CH_2$), 1.61 (s, 4H, $2CH_2$), 1.58 (m, 2H, $CH_2$), 1.25 (t, J=7.0 Hz, 3H, $CH_3$), 1.21 (s, 6H, $2CH_3$), 1.20 (s, 6H, $2CH_3$).

EXAMPLE 59

Preparation of compound **139a** according to Scheme X

**Ethyl 3-methyl-6-(3,4,6,7,8,9-hexahydro-6,6,9,9-tetramethyl-2H-benzo [g]quinolin-1-yl)hexa-2,4-dienoate** (structure **40**, where $R_1$, $R_2$, $R_3$, $R_4$, and $R_{19}$ are methyl; $R_5$, $R_6$, $R_7$, $R_8$, $R_9$, $R_{10}$ are hydrogen; $R_{15}$ is ethoxy; X, Y, and Z are carbon; m = 1, and n = 0).

The title compound was prepared from 2,3,5,6,7,8-hexahydro-5,5,8,8-tetramethylcyclopenta[b]naphthalen-1-one [U.S. patent 2,815,382 (1957)] in a manner similar to that described for compound **138a** of Example 57. Ester **139a** had $^1H$ NMR(400 MHz, $CDCl_3$) δ 6.88 (s, 1H, ArH), 6.51 (s, 1H, ArH), 6.29 (d, J=15.7 Hz, 1H, olefinic), 6.15 (dt, J=15.7, 5.6 Hz, 1H, olefinic), 5.72 (s, 1H, olefinic), 4.16 (q, J=7.0 Hz, 2H, $OCH_2$), 4.00 (d, J=5.6 Hz, 2H, allylic), 3.23 (br t, 2H, $NCH_2$), 2.73( br t, 2H, benzylic), 2.25 (s, 3H, $CH_3$), 1.96 (m, 2H, $CH_2$), 1.63 (s, 4H, $2CH_2$), 1.26 (t, J=7.0 Hz, 3H, $CH_3$), 1.24 (s, 6H, $2CH_3$), 1.23 (s, 6H, $2CH_3$).

**EXAMPLE 67**

Preparation of compound **143a** according to Scheme XII

Ethyl (2E,4E)-3-methyl-6-(1,2,3,4,7,8,9,10-octahydro-7,7,10,10-tetramethylbenzo[f]quinolin-4-yl)hexa-2 (structure **50**, where $R_1$, $R_2$, $R_3$, $R_4$, and $R_{19}$ are methyl; $R_9$ and $R_{10}$ are hydrogen; $R_{15}$ is ethoxy; X and Y are carbon; m=1, and n=0)

2,3,6,7,8,9-Hexahydro-6,6,9,9-tetramethyl- $^1H$-cyclopenta[a]naphthalen-3-one (prepared previously in Example 51) was coupled with $H_2NOH$-HCl to give the oxime which, without further purification, was subjected to LAH reduction to give 5,6,7,8-tetrahydro-5,5,8,8-tetramethyl(1,2)-naphthyl-[*b*]-piperidine: $^1H$ NMR (400 MHz, $CDCl_3$) δ 6.95 (d, J=8.7 Hz, 1H, ArH), 6.32 (d, J=8.7 Hz, 1H, ArH), 3.25 (t, J=6.3 Hz, 2H, $NCH_2$), 2.90 (t, J=6.0 Hz, 2H, benzylic), 1.87 (m, 2H, $CH_2$), 1.61 (m, 4H, $2CH_2$), 1.40 (s, 6H, $2CH_3$), 1.22 (s, 6H, $2CH_3$).

The above piperidine was then converted to the title compound in a manner similar to that of compound **89a** as described in Example 59. Compound **143a** had $R_f$=0.75 (20% ether in hexane); $^1H$ NMR (400 MHz, $CDCl_3$) δ 7.05 (d, J=8.7 Hz, 1H, ArH), 6.46 (d, J=8.7 Hz, 1H, ArH), 6.24 (d, J=15.8 Hz, 1H, olefinic), 6.13 (m, 1H, olefinic), 5.73 (s, $^1H$, olefinic), 4.15 (q, J=7.2 Hz, 2H, $OCH_2$), 3.94 (d, J=4.8 Hz, 2H, N-$CH_2$-allylic), 3.17 (t, J=6.3 Hz, 2H, N-$CH_2$), 2.90 (t, J=5.9 Hz, 2H, benzylic), 2.26 (s, 3H, $CH_3$), 1.97 (m, 2H, $CH_2$), 1.62 (m, 6H, $2CH_3$), 1.27 (t, J=7.2 Hz, 3H, $CH_3$), 1.24 (s, 6H, $2CH_3$).

**EXAMPLE 68**

Preparation of compound **143b** according to Scheme XII

**(2E,4E)-3-Methyl-6-(1,2,3,4,7,8,9,10-octahydro-7,7,10,10-tetramethylbenzo[f]quinolin-4-yl)hexa-2,4-dienoic acid** (structure **50**, where $R_1$, $R_2$, $R_3$, $R_4$, and $R_{19}$ are methyl; $R_9$ and $R_{10}$ are hydrogen; $R_{15}$ is hydroxy; X and Y are carbon; m=1, and n=0)

The title compound was prepared from compound **143a** using the standard hydrolysis conditions in Example 2. Compound **143b** had $R_f$=0.50 (50% ether in hexane); amorphous solid HRMS Calc. $C_{26}H_{33}O_2N$ 367.2511, found 367.2504; $^1H$ NMR (400 MHz, $CDCl_3$) δ 7.06 (d, J=8.7 Hz, 1H, ArH), 6.46 (d, J=8.7 Hz, 1H, ArH), 6.28 (d, J=15.7 Hz, 1H, olefinic), 6.22 (m, 1H, olefinic), 5.75 (s, 1H, olefinic), 3.95 (d, J=4.7 Hz, 2H, N-$CH_2$-allylic), 3.18 (t, J=6.2 Hz, 2H, N-$CH_2$), 2.90 (t, J=6.0 Hz, 2H, benzylic), 2.27 (s, 3H, $CH_3$), 1.90 (m, 2H, $CH_2$), 1.61 (m, 4H, $2CH_2$), 1.40 (s, 6H, $2CH_3$), 1.27 (s, 6H, $2CH_3$).

**Evaluation of Retinoid Receptor Subfamily Activity**

Utilizing the "cis-trans" or "co-transfection" assay described by Evans et al., Science, 240: 889-95 (May 13, 1988), the disclosure of which is herein incorporated by reference, the retinoid compounds of the present invention were tested and found to have strong, specific activity as either selective RAR agonists, selective RXR agonists, or as pan-agonist activators of both RAR and RXR receptors. This assay is described in further detail in U.S. Patent Nos. 4,981,784 and 5,071,773, the disclosures of which are incorporated herein by reference.

The co-transfection assay provides a method for identifying functional agonists which mimic, or antagonists which inhibit, the effect of native hormones, and quantifying their activity for responsive IR proteins. In this regard, the

co-transfection assay mimics an in vivo system in the laboratory. Importantly, activity in the co-transfection assay correlates very well with known in vivo activity, such that the co-transfection assay functions as a qualitative and quantitative predictor of a tested compounds in vivo pharmacology. See, e.g., T. Berger et al. 41 J. Steroid Biochem. Molec. Biol. 773 (1992), the disclosure of which is herein incorporated by reference.

In the co-transfection assay, a cloned cDNA for an IR (e.g., human RARα, RARβ, RXRγ) under the control of a constitutive promoter (e.g., the SV 40 promoter) is introduced by transfection (a procedure to induce cells to take up foreign genes) into a background cell substantially devoid of endogenous IRs. This introduced gene directs the recipient cells to make the IR protein of interest. A second gene is also introduced (co-transfected) into the same cells in conjunction with the IR gene. This second gene, comprising the cDNA for a reporter protein, such as firefly luciferase (LUC), controlled by an appropriate hormone responsive promoter containing a hormone response element (HRE). This reporter plasmid functions as a reporter for the transcription-modulating activity of the target IR. Thus, the reporter acts as a surrogate for the products (mRNA then protein) normally expressed by a gene under control of the target receptor and its native hormone.

The co-transfection assay can detect small molecule agonists or antagonists of target IRs. Exposing the transfected cells to an agonist ligand compound increases reporter activity in the transfected cells. This activity can be conveniently measured, e.g., by increasing luciferase production, which reflects compound-dependent, IR-mediated increases in reporter transcription. To detect antagonists, the co-transfection assay is carried out in the presence of a constant concentration of an agonist to the target IR (e.g., all-*trans* retinoic acid for RARα) known to induce a defined reporter signal. Increasing concentrations of a suspected antagonist will decrease the reporter signal (e.g., luciferase production). The co-transfection assay is therefore useful to detect both agonists and antagonists of specific IRs. Furthermore, it determines not only whether a compound interacts with a particular IR, but whether this interaction mimics (agonizes) or blocks (antagonizes) the effects of the native regulatory molecules on target gene expression, as well as the specificity and strength of this interaction.

The activity of the retinoid compounds of the present invention were evaluated utilizing the co-transfection assay according to the following illustrative Example.

**EXAMPLE 81**

**Co-transfection assay**

CV-1 cells (African green monkey kidney fibroblasts) were cultured in the presence of Dulbecco's Modified Eagle Medium (DMEM) supplemented with 10% charcoal resin-stripped fetal bovine serum then transferred to 96-well microtiter plates one day prior to transfection.

To determine RAR and/or RXR agonist activity of the compounds of the present invention, the CV-1 cells were transiently transfected by calcium phosphate coprecipitation according to the procedure of Berger et al., 41 *J. Steroid Biochem, Mol. Biol.,* 733 (1992) with the following receptor expressing plasmids: pRShRARα: Giguere *et al.,* 330 Nature, 624 (1987); pRShRARβ and pRShRARγ, Ishikawa *et al.,* 4 Mol. Endocrin., 837 (1990); pRShRXRα, Mangelsdorf *et al.,* 345 Nature, 224 (1990); and pRSmRXRβ and pRSmRXRγ, Mangelsdorf *et al.*, 6 Genes & Devel., 329 (1992), the disclosures of which are herein incorporated by reference. Each of these receptor expressing plasmids was co-transfected at a concentration of 5 ng/well, along with a basal reporter plasmid at 100 ng/well, the internal control plasmid pRS-β-Gal at 50 ng/well and filler DNA, pGEM at 45 ng/well.

The basal reporter plasmid D-MTV-LUC (Hollenberg and Evans, 55 Cell, 899 (1988). the disclosure of which is herein incorporated by reference) containing two copies of the TRE-palindromic response element described in Umesono *et al.,* 336 Nature, 262 (1988), the disclosure of which is herein incorporated by reference, was used in transfections for the RARs, and the reporter plasmid CRBPIIFKLUC, which contains an RXRE (retinoid X receptor response element, as described in Mangelsdorf *et al.*, 66 Cell, 555 (1991), the disclosure of which is herein incorporated by reference), was used in transfections for the RXRs. Each of these reporter plasmids contains the cDNA for firefly luciferase (LUC) under constitutive promoter containing the appropriate RAR or RXR response element. As noted above, pRS-β-Gal, coding for constitutive expression of E. coli β-galactosidase (β-Gal), was included as an internal control for evaluation of transfection efficiency and compound toxicity.

Six hours after transfection, media was removed and the cells were washed with phosphate-buffered saline (PBS). Media containing compounds of the present invention in concentrations ranging from $10^{-12}$ to $10^{-5}$ M were added to the cells. Similarly, the reference compounds all-*trans* retinoic acid (ATRA)(Sigma Chemical), a known RAR selective compound, and 9-*cis* retinoic acid (9-*cis)* (synthesized as described in Heyman *et al., Cell,* 68:397406 (1992)), a compound with known activity on RXRs, were added at similar concentrations to provide a reference point for analysis of the activity of the compounds of the present invention. Retinoid purity was established as greater than 99% by reverse phase high-performance liquid chromatography. Retinoids were dissolved in dimethylsulfoxide for use in the transcriptional activation assays. Three to four replicates were used for each sample.

After 40 hours, the cells were washed with PBS, lysed with a Triton X-100-based buffer and assayed for LUC and β-Gal activities using a luminometer or spectrophotometer, respectively. For each replicate, the normalized response (NR) was calculated as:

$$\text{LUC response}/\beta\text{-Gal rate}$$

where β-Gal rate = β-Gal•$1 \times 10^{-5}$/β-Gal incubation time.

The mean and standard error of the mean (SEM) of the NR were calculated. Data was plotted as the response of the compound compared to the reference compounds over the range of the dose-response curve. For the agonist compounds of the present invention, the effective concentration that produced 50% of the maximum response ($EC_{50}$) was quantified.

The potency (nM) of selected retinoid compounds of the present invention are in Table 1 below.

Table 1:

Potency (nM) of selected retinoid compounds of the present invention on RARα,β,γ and RXRα,β,γ, in comparison to the known RAR-active retinoid compound all-*trans* retinoic acid (**ATRA**) and RXR-active retinoid compound 9-*cis* retinoic acid (9-*cis*)

| | **RARα** | **RARβ** | **RARγ** | **RXRα** | **RXRβ** | **RXRγ** |
|---|---|---|---|---|---|---|
| **Cmpd. No.** | **Pot nM** | **Pot nM** | **Pot nM** | **Pot nM** | **Pot nM** | **Pot nM** |
| **135b** | 1166 | 53 | 47 | 2783 | 1979 | 2531 |
| **143b** | na | 466 | 1877 | 393 | 472 | 247 |
| **ATRA** | 436 | 78 | 19 | 1015 | 1211 | 961 |
| **9-*cis*** | 220 | 29 | 50 | 195 | 128 | 124 |

na = not active (potency of > 10,000 and/or efficacy of ≤ 20%)

## EXAMPLE 82

In addition to the cotransfection data of Example 81, the binding of selected compounds of the present invention to the RAR and RXR receptors was also investigated according to the methodology described in M.F., Boehm, et al., "Synthesis and Structure-Activity Relationships of Novel Retinoid X Receptor Selective Retinoids", 37 *J. Med. Chem.*, 2930 (1994); M.F. Boehm, et al., "Synthesis of High Specific Activity [$^3$H]-9-*cis* Retinoic Acid and Its Application for Identifying Retinoids with Unusual Binding Properties", 37 *J. Med. Chem.,* 408 (1994), and E.A. Allegretto, et al., "Characterization and Comparison of Hormone-Binding and Transactivation Properties of Retinoic Acid and Retinoid X Receptors Expressed in Mammalian Cells and Yeast", 268 *J. Biol. Chem.,* 22625 (1993), the disclosures of which are herein incorporated by reference.

Non-specific binding was defined as that binding remaining in the presence of 500 nM of the appropriate unlabelled compound. At the end of the incubation period, bound from free ligand were separated. The amount of bound tritiated retinoids was determined by liquid scintillation counting of an aliquot (700 mL) of the supernatant fluid or the hydroxylapatite pellet.

After correcting for non-specific binding, IC50 values were determined. The IC50 value is defined as the concentration of competing ligand needed to reduce specific binding by 50%. The $IC_{50}$ value was determined graphically from a log-logit plot of the data. The $K_i$ values were determined by application of the Cheng-Prussof equation to the $IC_{50}$ values, the labeled ligand concentration and the Kd of the labeled ligand.

## EXAMPLE 83

Yet another recognized measure of the retinoid activity of the compounds of the present invention is the ornithine decarboxylase assay, as originally described by Verma and Boutwell, 37 Cancer Research, 2196 (1977), the disclosure of which is herein incorporated by reference. In Verma & Boutwell original work using retinoic acid, it was established that ornithine decarboxylase (ODC) activity increased in relation to polyamine biosynthesis. In turn, it had previously been established that increases in polyamine biosynthesis is correlated with cellular proliferation. Thus, if ODC activity could be inhibited, cell hyperproliferation could be modulated. Although all causes of increased OCD activity are yet unknown, it is known that 12-0-tetradecanoylphorbor-13-acetate (TPA) induces ODC activity. Importantly, retinoic acid inhibits this induction of ODC by TPA.

An ODC assay essentially following the procedures set out in 35 Cancer Research, 1662 (1975), the disclosure of which is herein incorporated by reference, was used to demonstrate the inhibition of TPA induction of ODC by the compounds of the present invention.

**EXAMPLE 84**

The *in vitro* affect of selected compounds of the present invention on the recognized cancer cell lines, RPMI 8226, ME 180 and AML-193, obtained from the American Type Culture Collection (ATCC, Rockville, MD), was investigated.

RPMI 8226 is a human hematopoietic cell line obtained from the peripheral blood of a patient with multiple myeloma, and as such is a recognized model for multiple myelomas and related malignancies. Y. Matsuoka, G.E. Moore, Y. Yagi and D. Pressman, "Production of free light chains of immunoglobulin by a hematopoietic cell line derived from a patient with multiple myeloma", 125 *Proc. Soc. Exp. Biol. Med.,* 1246 (1967), the disclosure of which is herein incorporated by reference. The cells resemble the lymphoblastoid cells of other human lymphocyte cell lines and secretes $\lambda$-type light chains of immunoglobulin. RPMI 8226 cells were grown in RPMI medium (Gibco) supplemented with 10% fetal bovine serum, glutamine and antibiotics. The cells were maintained as suspension cultures grown at 37 °C in a humidified atmosphere of 5% $CO_2$ in air. The cells were diluted to a concentration of 1 x $10^5$/mL twice a week.

ME 180 is a human epidermoid carcinoma cell line derived from the cervix, and as such is a recognized model for squamous cell carcinomas and related malignancies. J.A. Sykes, J. Whitescarver, P. Jernstrom, J.F. Nolan and P. Byatt, "Some properties of a new epithelial cell line of human origin", 45 *MH-Adenoviridae J. Natl. Cancer Inst.,* 107 (1970), the disclosure of which is herein incorporated by reference. The tumor was a highly invasive squamous cell carcinoma with irregular cell clusters and no significant keratinization. ME 180 cells were grown and maintained in McCoy's 5a medium (Gibco) supplemented with 10% fetal bovine serum, glutamine and antibiotics. The cells were maintained as monolayer cultures grown at 37 °C in a humidified atmosphere of 5% $CO_2$ in air.

The AML-193 cell line was established from the blast cells of a patient with leukemia and was classified as M5 Acute Monocytic Leukemia, and as such is a recognized model for leukemias and related malignancies. G. Rovera, et al., 139 *J. Immunol.,* 3348 (1987), the disclosure of which is herein incorporated by reference. Over 75% of these cells are positive by immunofluorescence for the myelomonocytic antigen CS15. The cells were grown in Iscove's modified Dulbeccos's medium with 5 μg/mL transferring, 5 μg/mL insulin and 2 ng/mL rh GM-CSF. The cells were maintained as suspension cultures grown at 37 °C in a humidified atmosphere of 5% $CO_2$ in air. The cells were diluted to a concentration of 1 x $10^5$/mL twice a week.

Incorporation of $^3$H-Thymidine

Measurement of the level of radiolabeled thymidine incorporated into the above-identified cell lines provides a direct measurement of the antiproliferative properties of the compounds of the present invention. The method used for determination of the incorporation of radiolabeled thymidine was adapted from the procedure described by S. Shrivastav et al., "An in vitro assay procedure to test chemotherapeutic drugs on cells from human solid tumors", 40 *Cancer Res.,* 4438 (1980), the disclosure of which is herein incorporated by reference. RPMI 8226 or AML-193 cells were plated in a 96 well round bottom microtiter plate (Costar) at a density of 1,000 cells/well. To appropriate wells, retinoid test compounds were added at the final concentrations indicated for a final volume of 150 μL/well. The plates were incubated for 96 hours at 37°C in a humidified atmosphere of 5% $CO_2$ in air. Subsequently, 1 μCi of [5'-$^3$H]-thymidine (Amersham, U.K, 43 Ci/mmol specific activity) in 25 μL culture medium was added to each well and the cells were incubated for an additional six hours. The cultures were further processed as described below.

ME 180 cells, harvested by trypsinization were plated in a 96 well flat bottom microtiter plate (Costar) at a density of 2,000 cells/well. The cultures were treated as described above for RPMI 8226 with the following exceptions. After incubation, the supernatant was carefully removed, and the cells were washed with a 0.5 mM solution of thymidine in phosphate buffered saline. ME 180 cells were briefly treated with 50 μL of 2.5% trypsin to dislodge the cells from the plate. Both cell lines were then processed as follows: the cellular DNA was precipitated with 10% trichloroacetic acid onto glass fiber filter mats using a SKATRON multi-well cell harvester (Skatron Instruments, Sterling VA). Radioactivity incorporated into DNA, as a direct measurement of cell growth, was measured by liquid scintillation counting. The mean disintegrations per minute of incorporated thymidine from triplicate wells was determined.

Viability

Selected compounds of the present invention were also measured to determine their cytotoxicity on the above-identified cell lines. The procedure used was identical, with only slight modifications, to the assay described in T.

Mosmann, "Rapid colorimetric assay for cellular growth and survival: application to proliferation and cytotoxicity assays", 65 *J. Immunol. Meth.,* 55 (1983), the disclosure of which is herein incorporated by reference. RPMI 8226 or AML-193 cells were plated in a 96 well round bottom microtiter plate (Costar) at a density of 1,000 cells/well. To appropriate wells, retinoid test compounds were added at the final concentrations indicated for a final volume of 150 µL/well. The plates were incubated for 96 hours at 37 °C in a humidified atmosphere of 5% $CO_2$ in air. Subsequently, 15 µL of a filter sterilized tetrazolium dye in phosphate buffered saline (Promega, Madison, WI) was added to each well and the cells were incubated for an additional four hours. Subsequent manipulations of the cultures were as described below. ME 180 cells, harvested by trypsinization were plated in a 96 well flat bottom microtiter plate (Costar) at a density of 2,000 cells/well. The cultures were treated as described above for RPMI 8226.

After the four hours incubation, 100 µL of a solubilization/stop solution was added to each well (Promega, Madison, WI). The plates were allowed to stand overnight at 37 °C in the humidified atmosphere. The absorbance at 570 - 600 nm wavelength was recorded for each well using a Biomek ELISA plate reader (Beckman Instruments).

**EXAMPLE 85**

The following examples provide illustrative pharmacological composition formulations: Hard gelatin capsules are prepared using the following ingredients:

| | Quantity (mg/capsule) |
|---|---|
| Compound **101b** | 140 |
| Starch, dried | 100 |
| Magnesium stearate | 10 |
| Total | 250 mg |

The above ingredients are mixed and filled into hard gelatin capsules in 250 mg quantities.

A tablet is prepared using the ingredients below:

| | Quantity (mg/tablet) |
|---|---|
| Compound **101b** | 140 |
| Cellulose, microcrystalline | 200 |
| Silicon dioxide, fumed | 10 |
| Stearic acid | 10 |
| Total | 360 mg |

The components are blended and compressed to form tablets each weighing 360 mg Tablets, each containing 60 mg of active ingredient, are made as follows:

| | Quantity (mg/tablet) |
|---|---|
| Compound **101b** | 60 |
| Starch | 45 |
| Cellulose, microcrystalline | 35 |
| Polyvinylpyrrolidone (PVP) (as 10% solution in water) | 4 |
| Sodium carboxymethyl starch (SCMS) | 4.5 |
| Magnesium stearate | 0.5 |
| Talc | 1.0 |
| Total | 150 mg |

The active ingredient, starch, and cellulose are passed through a No. 45 mesh U.S. sieve and mixed thoroughly. The solution of PVP is mixed with the resultant powders, which are then passed through a No. 14 mesh U.S. sieve. The granules so produced are dried at 50°C and passed through a No. 18 mesh U.S. sieve. The SCMS, magnesium stearate, and talc, previously passed through a No. 60 mesh U.S. sieve, are then added to the granules which, after mixing, are compressed on a tablet machine to yield tablets each weighing 150 mg.

Suppositories, each containing 225 mg of active ingredient, may be made as follows:

| | |
|---|---|
| Compound **101b** | 225 mg |
| Saturated fatty acid glycerides | 2.000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of normal 2g capacity and allowed to cool An intravenous formulation may be prepared as follows:

| | |
|---|---|
| Compound **101b** | 100 mg |
| Isotonic saline | 100 mg |
| Glycerol | 100 ml |

The compound is dissolved in the glycerol and then the solution is slowly diluted with isotonic saline. The solution of the above ingredients is then administered intravenously at a rate of 1 ml per minute to a patient.

While in accordance with the patent statutes, description of the preferred embodiments and processing conditions have been provided, the scope of the invention is not to be limited thereto or thereby. Various modifications and alterations of the present invention will be apparent to those skilled in the art without departing from the scope and spirit of the present invention.

Consequently, for an understanding of the scope of the present invention, reference is made to the following claims.

## Claims

**1.** A tricyclic compound of the formula:

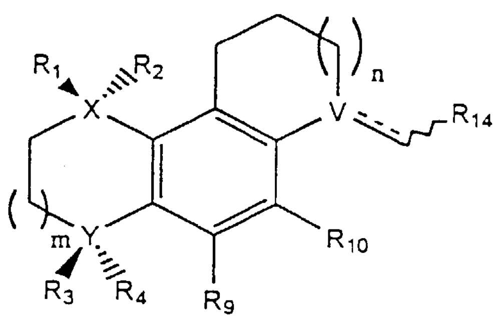

(III)

wherein,

$R_1$ through $R_4$ each independently are hydrogen, a $C_1$ - $C_6$ alkyl, or a $C_7$ - $C_{15}$ arylalkyl;
$R_9$ and $R_{10}$ each independently are hydrogen, a $C_1$ - $C_6$ alkyl, F, Cl, Br, $NR_{11}R_{12}$, $NO_2$ or $OR_{13}$, where $R_{11}$ and $R_{12}$ each independently are hydrogen, a $C_1$ - $C_8$ alkyl, a $C_7$ - $C_{15}$ arylalkyl, a $C_1$ - $C_8$ acyl, provided that only one of $R_{11}$ or $R_{12}$ can be acyl, or $R_{11}$ and $R_{12}$ taken together are a $C_3$ - $C_6$ cycloalkyl, and where $R_{13}$ is hydrogen or a $C_1$ - $C_8$ alkyl or a $C_7$ - $C_{15}$ arylalkyl;
$R_{14}$ represents:

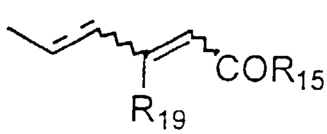
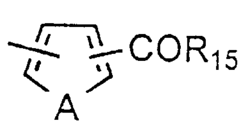
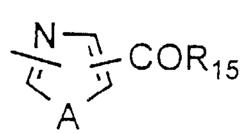

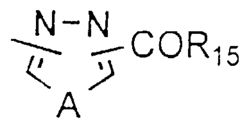

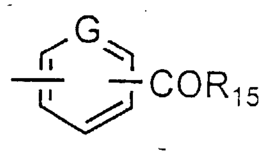

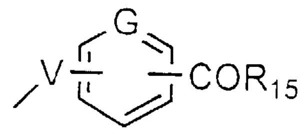

where $R_{15}$ is $OR_{16}$ or $NR_{17}R_{18}$, with $R_{16}$ being hydrogen, a $C_1$ - $C_6$ alkyl or a $C_7$ - $C_{15}$ arylalkyl, and with $R_{17}$ and $R_{18}$ each independently being hydrogen, a $C_1$ - $C_6$ alkyl, a $C_7$ - $C_{15}$ arylalkyl, aryl, ortho-, meta-, or para-substituted hydroxyaryl, or taken together are a $C_3$ - $C_6$ cycloalkyl, provided that $R_{18}$ must be hydrogen when $R_{17}$ is aryl or hydroxyaryl, $R_{19}$ is a $C_1$ - $C_5$ alkyl, and A is O, S or $NR_{20}$, where $R_{20}$ is a hydrogen, $C_1$ - $C_6$ alkyl or a $C_7$ - $C_{15}$ arylalkyl;

X and Y each independently represent C, O, S, N, SO or $SO_2$, provided, however, that when X or Y are O, S, SO or $SO_2$, then either $R_1$ and $R_2$ or $R_3$ and $R_4$ respectively do not exist, and further provided, that when X or Y is N, then one each of $R_1$ and $R_2$ or $R_3$ and $R_4$ respectively, do not exist;

V is C or N, provided, however, that when V is N, then no double bond exists adjacent to V;

G is C or N;

m is 0 or 1 carbon atoms;

n is 0, 1 or 2 carbon atoms;

the dashed lines in the structures represent optional double bonds; and

the wavy lines represent olefin bonds that are either in the *cis (Z)* or *trans* (E) configuration.

**2.** A compound according to claim 1, selected from the group consisting of Ethyl(2E,4E)-3-methyl-6-[(E)-2,3,6,7,8,9-hexahydro-6,6,9,9-tetramethyl-1H-cyclopenta[a]naphthalen-3-ylidene]hexa-2,4-dienoate; (2E,4E)-3- methyl-6[(E)-2,3,6,7,8,9-hexahydro-6,6,9,9-tetramethyl-1H-cyclopenta [a]naphthalen-3-ylidene]hexa-2,4-dienoic acid; ethyl (2E,4E)-3-methyl-6-(2,3,6,7,8,9-hexahydro-6,6,9,9-tetramethyl-1H-cyclopenta[a]naphthalen-3-yl]hexa-2,4-dienoate; (2E,4E)-3-methyl-6-(2,3,6,7,8,9-hexahydro-6,6,9,9-tetramethyl-1H-cyclopenta [a]naphthalen-3-yl)hexa-2,4-dienoic acid; ethyl (2E,4E)-3-methyl-6-[(Z)1,2,3,4,7,8,9-heptahydro-7,7,9,9-tetramethylcydopenta[f]naphthalen-4-ylidene]hexa-2,4-dienoate; (2E,4E)-3-methyl-6-[(Z)-1,2,3,4,7,8,9-heptahydro-7,7,9,9-tetramethylcyclopenta [f]naphthalen-4-ylidene] hexa-2,4-dienoic acid; ethyl (2E,4E)-3-methyl-6-(1,2,3,4,7,8,9,10-octahydro-7,7,10,10-tetramethylbenzo[f]quinolin-4-yl)hexa-2,4-dienoate; and (2E,4E)-3-methyl-6-(1,2,3,4,7,8,9,10-octahydro-7,7,10,10-tetramethylbenzo[f]quinolin-4-yl)hexa-2,4-dienoic acid.

**3.** A pharmaceutical composition comprising a compound of claim 1 and a pharmaceutically acceptable carrier.

**4.** A pharmaceutical composition according to claim 3, wherein the composition is formulated for oral, topical, intravenous, suppository or parental administration.

**5.** A pharmaceutical composition according to claim 3, which is in the form of a dosage unit containing from about 1µg/kg of body weight to about 500 mg/kg of body weight of the compound.

**6.** A pharmaceutical composition according to claim 3, which is in the form of a dosage unit containing from about 10µg/kg of body weight to about 250 mg/kg of body weight of the compound.

**7.** A pharmaceutical composition according to claim 3, which is in the form of a dosage unit containing from about 20µg/kg of body weight to about 100 mg/kg of body weight of the compound.

**8.** Use of a retinoid compound according to claim 1 for the manufacture of a medicament for affecting RAR and/or RXR activity by *in vivo* administration of the compound.

**9.** Use of a retinoid compound according to claim 1 for the manufacture of a medicament for modulating processes mediated by RAR and/or RXR receptors by administering to a patient an amount of the compound, said compound being effective to modulate one or more processes mediated by RAR and/or RXR receptors.

**10.** Use of a retinoid compound according to claim 1 for the manufacture of a medicament for the treatment of a patient requiring retinoid therapy by administering to the patient a pharmaceutically effective amount of the compound.

**11.** Use according to claim 10, wherein the compound is effective in treating skin-related diseases and conditions, cancerous and pre-cancerous conditions, diseases of the eye, cardiovascular diseases, inflammatory diseases, neurodegenerative diseases, diseases involving modulation of apoptosis, diseases of the immune system, improper pituitary function, diseases involving human papilloma virus, wound healing or restoration of hair growth.

**12.** Use of a pharmaceutical composition according to claim 3 for the manufacture of a medicament for treating a patient requiring retinoid therapy by administering to the patient a pharmaceutically effective amount of the composition.

**13.** Use according to claim 12, wherein the composition is effective in treating skin-related diseases and conditions, cancerous and pre-cancerous conditions, diseases of the eye, cardiovascular diseases, inflammatory diseases, neurodegenerative diseases, diseases involving modulation of apoptosis, diseases of the immune system, improper pituitary function, diseases involving human papilloma virus, wound healing or restoration of hair growth.

**14.** A method for determining the presence of one or more RAR and/or RXR receptors in a sample comprising combining a compound according to claim 1 with the sample containing one or more unknown retinoid receptors, and determining whether said compound binds to a receptor in the sample.

**15.** A ligand-retinoid receptor complex formed by the binding of a compound according to claim 1 to a RAR and/or RXR receptor.

**16.** A method of purifying retinoid receptors comprising combining a compound according to claim 1 with a sample containing RAR and/or RXR receptors, allowing said compound to bind said receptors, and separating out the bound combination of said compound and said RAR and/or RXR receptors.

**Patentansprüche**

**1.** Eine tricyclische Verbindung der Formel:

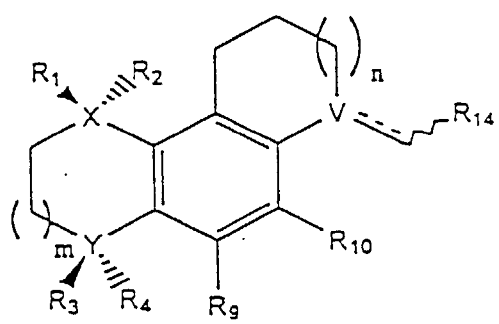

(III)

worin

$R_1$ bis $R_4$ jeweils unabhängig voneinander Wasserstoff, eine $C_1$-$C_6$-Alkylgruppe oder eine $C_7$-$C_{15}$-Arylalkylgruppe sind;

$R_9$ und $R_{10}$ jeweils unabhängig voneinander Wasserstoff, eine $C_1$-$C_6$-Alkylgruppe, F, Cl, Br, $NR_{11}R_{12}$, $NO_2$ oder $OR_{13}$ sind, wobei $R_{11}$ und $R_{12}$ jeweils unabhängig voneinander Wasserstoff, eine $C_1$-$C_8$-Alkylgruppe, eine $C_7$-$C_{15}$-Arylalkylgruppe, eine $C_1$-$C_8$-Acylgruppe sind, vorausgesetzt, daß nur einer von $R_{11}$ und $R_{12}$ eine Acylgruppe sein kann, oder $R_{11}$ und $R_{12}$ zusammengenommen eine $C_3$-$C_6$-Cycloalkylgruppe sind, und wobei $R_{13}$ Wasserstoff oder eine $C_1$-$C_8$-Alkylgruppe oder eine $C_7$-$C_{15}$-Arylalkylgruppe ist;

$R_{14}$ für

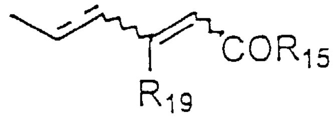
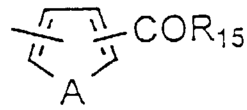
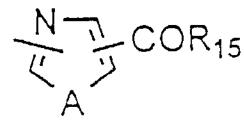

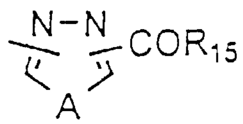
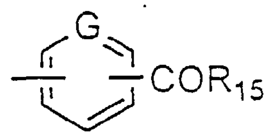
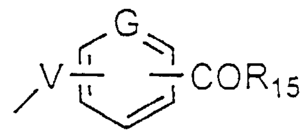

steht, wobei $R_{15}$ $OR_{16}$ oder $NR_{17}R_{18}$ ist, wobei $R_{16}$ Wasserstoff, eine $C_1$-$C_6$-Alkylgruppe oder eine $C_7$-$C_{15}$-Arylalkylgruppe ist und wobei $R_{17}$ und $R_{18}$ jeweils unabhängig voneinander Wasserstoff, eine $C_1$-$C_6$-Alkylgruppe, eine $C_7$-$C_{15}$-Arylalkylgruppe, eine Arylgruppe, eine ortho-, meta- oder para-substituierte Hydroxyarylgruppe sind oder zusammengenommen eine $C_3$-$C_6$-Cycloalkylgruppe sind, vorausgesetzt, daß $R_{18}$ Wasserstoff sein muß, wenn $R_{17}$ eine Arylgruppe oder eine Hydroxyarylgruppe ist, $R_{19}$ eine $C_1$-$C_5$-Alkylgruppe ist und A O, S oder $NR_{20}$ ist, wobei $R_{20}$ Wasserstoff, eine $C_1$-$C_6$-Alkylgruppe oder eine $C_7$-$C_{15}$-Arylalkylgruppe ist;

X und Y jeweils unabhängig voneinander für C, O, S, N, SO oder $SO_2$ stehen, vorausgesetzt jedoch, daß wenn X und Y 0, S, SO oder $SO_2$ sind, dann entweder $R_1$ und $R_2$ bzw. $R_3$ und $R_4$ nicht existieren, und weiterhin vorausgesetzt, daß wenn X oder Y N ist, dann jeweils einer von $R_1$ und $R_2$ bzw. $R_3$ und $R_4$ nicht existiert;

V C oder N ist, vorausgesetzt jedoch, daß wenn V N ist, dann keine Doppelbindung neben V existiert;

G C oder N ist;

m 0 oder 1 Kohlenstoffatom bedeutet;

n 0, 1 oder 2 Kohlenstoffatome bedeutet;

die gestrichelten Linien in den Strukturen optionale Doppelbindungen darstellen; und

die welligen Linien Olefinbindungen darstellen, die entweder in der *cis* (Z)- oder *trans* (E)-Konfiguration vorliegen.

**2.** Eine Verbindung gemäß Anspruch 1, die ausgewählt ist aus der Gruppe bestehend aus Ethyl(2E,4E)-3-methyl-6-[(E)-2,3,6,7,8,9-hexahydro-6,6,9,9-tetramethyl-1H-cyclopenta[a]naphthalin-3-yliden]hexa-2,4-dienoat; (2E,4E)-3-Methyl-6-[(E)-2,3,6,7,8,9-hexahydro-6,6,9,9-tetramethyl-1H-cyclopenta[a]naphthalin-3-yliden]hexa-2,4-diensäure; Ethyl(2E,4E)-3-methyl-6-(2,3,6,7,8,9-hexahydro-6,6,9,9-tetramethyl-1H-cyclopenta[a]naphthalin-3-yl]hexa-2,4-dienoat; (2E,4E)-3-Methyl-6-(2,3,6,7,8,9-hexahydro-6,6,9,9-tetramethyl-1H-cyclopenta[a]naphthalin-3-yl)hexa-2,4-diensäure; Ethyl(2E,4E)-3-methyl-6-[(Z)-1,2,3,4,7,8,9-heptahydro-7,7,9,9-tetramethylcyclopenta[f]naphthalin-4-yliden]hexa-2,4-dienoat; (2E,4E)-3-Methyl-6-[(Z)-1,2,3,4,7,8,9-heptahydro-7,7,9,9-tetramethylcyclopenta[f]naphthalin-4-yliden]hexa-2,4-diensäure; Ethyl(2E,4E)-3-methyl-6-(1,2,3,4,7,8,9,10-octahydro-7,7,10,10-tetramethylbenzo[f]chinolin-4-yl)hexa-2,4-dienoat; und (2E,4E)-3-Methyl-6-(1,2,3,4,7,8,9,10-octahydro-7,7,10,10-tetramethylbenzo[f]chinolin-4-yl)hexa-2,4-diensäure.

**3.** Eine pharmazeutische Zusammensetzung, welche eine Verbindung nach Anspruch 1 und einen pharmazeutisch annehmbaren Träger umfaßt.

**4.** Eine pharmazeutische Zusammensetzung gemäß Anspruch 3, wobei die Zusammensetzung für eine orale, topische, intravenöse, suppositorische oder parenterale Verabreichung formuliert ist.

**5.** Eine pharmazeutische Zusammensetzung gemäß Anspruch 3, welche in Form einer Dosierungseinheit vorliegt,

die von ca. 1 µg/kg Körpergewicht bis ca. 500 mg/kg Körpergewicht der Verbindung enthält.

**6.** Eine pharmazeutische Zusammensetzung gemäß Anspruch 3, welche in Form einer Dosierungseinheit vorliegt, die von ca. 10 µg/kg Körpergewicht bis ca. 250 mg/kg Körpergewicht der Verbindung enthält.

**7.** Eine pharmazeutische Zusammensetzung gemäß Anspruch 3, welche in Form einer Dosierungseinheit vorliegt, die von ca. 20 µg/kg Körpergewicht bis ca. 100 mg/kg Körpergewicht der Verbindung enthält.

**8.** Verwendung einer Retinoid-Verbindung gemäß Anspruch 1 zur Herstellung eines Medikaments zur Beeinflussung der RAR- und/oder RXR-Aktivität durch eine *in vivo*-Verabreichung der Verbindung.

**9.** Verwendung einer Retinoid-Verbindung gemäß Anspruch 1 zur Herstellung eines Medikaments zum Modulieren von Prozessen, die durch RAR- und/oder RXR-Rezeptoren vermittelt werden, indem einem Patienten eine Menge der Verbindung verabreicht wird, wobei die Verbindung wirksam ist, um einen oder mehrere der Prozesse zu modulieren, die durch RAR- und/oder RXR-Rezeptoren vermittelt werden.

**10.** Verwendung einer Retinoid-Verbindung gemäß Anspruch 1 zur Herstellung eines Medikaments zur Behandlung eines Patienten, welcher eine Retinoid-Therapie benötigt, durch ein Verabreichen einer pharmazeutisch wirksamen Menge der Verbindung an den Patienten.

**11.** Verwendung gemäß Anspruch 10, wobei die Verbindung wirksam ist bei der Behandlung von Krankheiten und Zuständen, die mit der Haut zusammenhängen, kanzerösen und präkanzerösen Zuständen, Augenkrankheiten, kardiovaskulären Krankheiten, entzündlichen Krankheiten, neurodegenerativen Krankheiten, Krankheiten, die mit einer Modulation der Apoptosis verbunden sind, Krankheiten des Immunsystems, einer gestörten Hypophysenfunktion, Krankheiten, an denen das menschliche Papilloma-Virus beteiligt ist, der Wundheilung oder der Wiederherstellung des Haarwachstums.

**12.** Verwendung einer pharmazeutischen Zusammensetzung gemäß Anspruch 3 zur Herstellung eines Medikaments zur Behandlung eines Patienten, welcher eine Retinoid-Therapie benötigt, durch ein Verabreichen einer pharmazeutisch wirksamen Menge der Zusammensetzung an den Patienten.

**13.** Verwendung gemäß Anspruch 12, wobei die Zusammensetzung wirksam ist bei der Behandlung von Krankheiten und Zuständen, die mit der Haut zusammenhängen, kanzerösen und präkanzerösen Zuständen, Augenkrankheiten, kardiovaskulären Krankheiten, entzündlichen Krankheiten, neurodegenerativen Krankheiten, Krankheiten, die mit einer Modulation der Apoptosis verbunden sind, Krankheiten des Immunsystems, einer gestörten Hypophysenfunktion, Krankheiten, an denen das menschliche Papilloma-Virus beteiligt ist, der Wundheilung oder der Wiederherstellung des Haarwachstums.

**14.** Ein Verfahren zum Feststellen des Vorliegens von einem oder mehreren RAR- und/oder RXR-Rezeptoren in einer Probe, welches ein Vereinigen einer Verbindung gemäß Anspruch 1 mit der Probe, welche einen oder mehrere unbekannte Retinoid-Rezeptoren enthält, und ein Feststellen, ob die Verbindung an einen Rezeptor in der Probe bindet, umfaßt.

**15.** Ein Liganden-Retinoid-Rezeptor-Komplex, welcher durch die Bindung von einer Verbindung gemäß Anspruch 1 an einen RAR- und/oder RXR-Rezeptor gebildet wurde.

**16.** Ein Verfahren zur Reinigung eines Retinoid-Rezeptors, welches ein Vereinigen einer Verbindung gemäß Anspruch 1 mit einer Probe, welche RAR- und/oder RXR-Rezeptoren enthält, ein Bindenlassen der Verbindung an die Rezeptoren und ein Abtrennen der verbundenen Kombination der Verbindung und der RAR- und/oder RXR-Rezeptoren umfaßt.

**Revendications**

**1.** Composé tricyclique représenté par la formule :

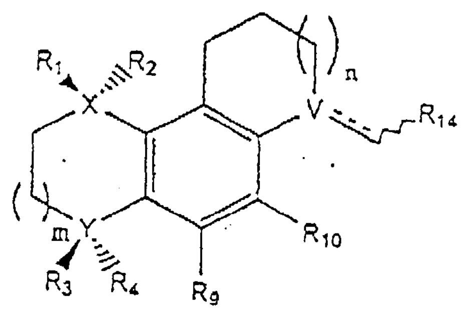

(III)

dans laquelle :

$R_1$ à $R_4$ sont chacun indépendamment un atome d'hydrogène, un groupe alkyle en $C_{1-6}$ ou un groupe arylalkyle en $C_{7-15}$;
$R_9$ et $R_{10}$ sont chacun indépendamment un atome d'hydrogène, un groupe alkyle en $C_{1-6}$, F, Cl, Br, $NR_{11}R_{12}$, $NO_2$ ou $OR_{13}$, où $R_{11}$ et $R_{12}$ sont chacun indépendamment un atome d'hydrogène, un groupe alkyle en $C_{1-8}$, un groupe arylalkyle en $C_{7-15}$, un groupe acyle en $C_{1-8}$, à condition que l'un seulement de $R_{11}$ ou $R_{12}$ soit un groupe acyle, ou bien $R_{11}$ et $R_{12}$ pris ensemble forment un groupe cycloalkyle en $C_{3-6}$, et où $R_{13}$ est un atome d'hydrogène, un groupe alkyle en $C_{1-8}$ ou un groupe arylalkyle en $C_{7-15}$ ;
$R_{14}$ représente :

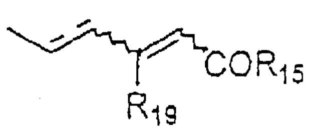
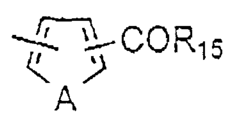
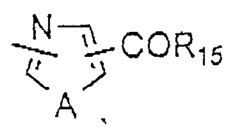

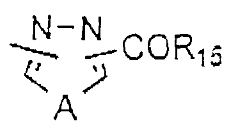
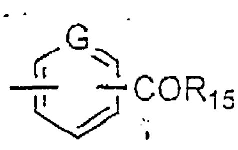
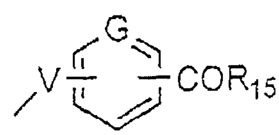

où :
$R_{15}$ est $OR_{16}$ ou $NR_{17}R_{18}$, où $R_{16}$ est un atome d'hydrogène, un groupe alkyle en $C_{1-6}$ ou un groupe arylalkyle en $C_{7-15}$, et où $R_{17}$ et $R_{18}$ sont chacun indépendamment un atome d'hydrogène, un groupe alkyle en $C_{1-6}$, un groupe arylalkyle en $C_{7-15}$, un groupe aryle, hydroxyaryle ortho-, méta- ou para-substitué, ou pris ensemble, ils forment un groupe cycloalkyle en $C_{3-6}$, à condition que $R_{18}$ soit un atome d'hydrogène lorsque $R_{17}$ est un groupe aryle ou hydroxyaryle, $R_{19}$ est un groupe alkyle en $C_{1-5}$, et A est O, S ou $NR_{20}$, où $R_{20}$ est un atome d'hydrogène, un groupe alkyle en $C_{1-6}$ ou un groupe arylalkyle en $C_{7-15}$ ;
X et Y sont chacun indépendamment C, O, S, N, SO ou $SO_2$, à condition, cependant, que lorsque X ou Y est O, S, SO ou $SO_2$, alors l'un ou l'autre des $R_1$ et $R_2$ ou des $R_3$ et $R_4$, respectivement, n'existe pas, et à condition de plus que lorsque X ou Y est N, alors l'un des $R_1$ et $R_2$ ou l'un des $R_3$ et $R_4$, respectivement, n'existe pas ;
V est C ou N, à condition, cependant, que lorsque V est N, il n'y ait aucune double liaison adjacente à V ;
G est C ou N ;
m est 0 ou atome de carbone ;
n est 0, 1 ou 2 atomes de carbone ;
les lignes tiretées dans les structures représentent des doubles liaisons éventuelles ; et
les lignes ondulées représentent des liaisons oléfiniques qui sont en configuration soit *cis* (Z), soit *trans* (E).

**2.** Composé suivant la revendication 1, choisi dans le groupe consistant en :

(2E, 4E)-3-méthyl-6-[(E)-2,3,6,7,8,9-hexahydro-6,6,9,9-tétraméthyl-1H-cyclopenta-[a]-naphtalèn-3-ylidène]-hexa-2,4-diénoate d'éthyle ;

acide (2E, 4E)-3-méthyl-6-[(E )-2,3,6,7,8,9-hexahydro-6,6,9,9-tétraméthyl-1H-cyclopenta-[a]-naphtalèn-3-ylidène]-hexa-2,4-diénoïque ;
(2E, 4E)-3-méthyl-6-(2,3,6,7,8,9-hexahydro-6,6,9,9-tétraméthyl-1H-cyclopenta-[a]-naphtalèn-3-yl)-hexa-2,4-diénoate d'éthyle ;
acide (2E, 4E)-3-méthyl-6-(2,3,6,7,8,9-hexahydro-6,6,9,9-tétraméthyl-1H-cyclopenta-[a]-naphtalèn-3-yl)-hexa-2,4-diénoïque ;
(2E, 4E)-3-méthyl-6-[(Z)-1,2,3,4,7,8,9-heptahydro-7,7,9,9-tétraméthyl-cyclopenta-[f]-naphtalèn-4-ylidène]-hexa-2,4-diénoate d'éthyle ;
acide (2E, 4E)-3-méthyl-6-[(Z)-1,2,3,4,7,8,9-heptahydro-7,7,9,9-tétraméthyl-cyclopenta-[f]-naphtalèn-4-ylidène]-hexa-2,4-diénoïque ;
(2E, 4E)-3-méthyl-6-(1,2,3,4,7,8,9,10-octahydro-7,7,10,10-tétraméthyl-benzo-[f]-quinoléin-4-yl)-hexa-2,4-diénoate d'éthyle ; et
acide (2E, 4E)-3-méthyl-6-(1,2,3,4,7,8,9,10-octahydro-7,7,10,10-tétraméthyl-benzo-[f]-quinoléin-4-yl)-hexa-2,4-diénoïque.

**3.** Composition pharmaceutique comprenant un composé suivant la revendication 1 et un support acceptable du point de vue pharmaceutique.

**4.** Composition pharmaceutique suivant la revendication 3, dans laquelle la composition est formulée pour l'administration orale, locale, intraveineuse, rectale ou parentérale.

**5.** Composition pharmaceutique suivant la revendication 3, qui est sous la forme d'une dose unitaire contenant d'environ 1 µg/kg de poids corporel à environ 500 mg/kg de poids corporel du composé.

**6.** Composition pharmaceutique suivant la revendication 3, qui est sous la forme d'une dose unitaire contenant d'environ 10 µg/kg de poids corporel à environ 250 mg/kg de poids corporel du composé.

**7.** Composition pharmaceutique suivant la revendication 3, qui est sous la forme d'une dose unitaire contenant d'environ 20 µg/kg de poids corporel à environ 100 mg/kg de poids corporel du composé.

**8.** Utilisation d'un composé rétinoïde suivant la revendication 1 pour la fabrication d'un médicament destiné à exercer un effet sur l'activité RAR et/ou RXR par administration *in vivo* du composé.

**9.** Utilisation d'un composé rétinoïde suivant la revendication 1 pour la fabrication d'un médicament destiné à moduler des processus médiés par les récepteurs RAR et/ou RXR par administration au patient d'une quantité du composé, ledit composé étant efficace pour moduler un ou plusieurs processus médiés par les récepteurs RAR et/ou RXR.

**10.** Utilisation d'un composé rétinoïde suivant la revendication 1 pour la fabrication d'un médicament destiné au traitement d'un patient nécessitant un traitement par un rétinoïde par administration au patient d'une quantité efficace du point de vue pharmaceutique du composé.

**11.** Utilisation suivant la revendication 10, dans laquelle le composé est efficace dans le traitement d'affections cutanées et d'états apparentés, d'états cancéreux et précancéreux, d'affections de l'oeil, d'affections cardiovasculaires, d'affections inflammatoires, de maladies neurodégénératives, d'affections impliquant une modulation de l'apoptose, de maladies du système immun, d'un dysfonctionnement de la fonction hypophysaire, de maladies impliquant le virus du papillome humain, pour la guérison des plaies ou la restauration de la pousse des cheveux.

**12.** Utilisation d'une composition pharmaceutique suivant la revendication 3 pour la fabrication d'un médicament destiné au traitement d'un patient nécessitant un traitement par un rétinoïde par administration au patient d'une quantité efficace du point de vue pharmaceutique de la composition.

**13.** Utilisation suivant la revendication 12, dans laquelle la composition est efficace dans le traitement d'affections cutanées et d'états apparentés, d'états cancéreux et pré-cancéreux, d'affections de l'oeil, d'affections cardiovasculaires, d'affections inflammatoires, de maladies neurodégénératives, d'affections impliquant une modulation de l'apoptose, de maladies du système immun, d'un dysfonctionnement de la fonction hypophysaire, de maladies impliquant le virus du papillome humain, pour la guérison des plaies ou la restauration de la pousse des cheveux.

**14.** Procédé pour déterminer la présence d'un ou plusieurs récepteurs RAR et/ou RXR dans un échantillon, comprenant l'association d'un composé suivant la revendication 1 avec l'échantillon contenant un ou plusieurs récepteurs rétinoïdes inconnus, et la déterminaison du fait que ledit composé se lie ou non à un récepteur dans l'échantillon.

**15.** Complexe ligand-récepteur rétinoïde formé par la liaison d'un composé suivant la revendication 1 à un récepteur RAR et/ou RXR.

**16.** Procédé pour purifier des récepteurs rétinoïdes comprenant la combinaison d'un composé suivant la revendication 1 avec un échantillon contenant des récepteurs RAR et/ou RXR, le maintien de celle-ci pendant un temps suffisant pour que ledit composé se lie auxdits récepteurs, et la séparation de la combinaison liée dudit composé et desdits récepteurs RAR et/ou RXR.